# EUROPÄISCHE PATENTSCHRIFT

(11) **EP 4 366 665 B1**
(45) Veröffentlichungstag und Bekanntmachung des Hinweises auf die Patenterteilung: **09.12.2026**
(21) Anmeldenummer: 22748312.0
(22) Anmeldetag: 08.07.2022
(51) Int. Cl.: A61F 2/74, A61F 2/70, A61F 2/64, A61F 2/58, A61F 5/01, A61F 2/66, A61F 2/50

(54) **ORTHOPÄDIETECHNISCHE GELENKEINRICHTUNG**
ORTHOPEDIC JOINT DEVICE
DISPOSITIF D'ARTICULATION ORTHOPÉDIQUE

(30) Priorität: 09.07.2021 DE 102021117839
(43) Veröffentlichungstag der Anmeldung: 15.05.2024
(73) Patentinhaber: Otto Bock Healthcare Products GmbH, 1110 Wien (AT)
(72) Erfinder: AUBERGER, Roland, 1140 Wien (AT); PICKERILL, Thomas West, 1030 Wien (AT); BREUER-RUESCH, Christian, 1230 Wien (AT)
(74) Vertreter: Gramm, Lins & Partner Patent- und Rechtsanwälte PartGmbB
(86) Internationale Anmeldenummer: PCT/EP2022/069085
(87) Internationale Veröffentlichungsnummer: WO 2023/281062

(56) Entgegenhaltungen:
- EP-B1- 1 996 128
- EP-B1- 2 502 607
- DE-A1- 102018 217 497
- DE-C- 334 426
- US-B1- 6 517 503

## Beschreibung

Orthopädietechnische Gelenkeinrichtungen sind insbesondere Orthesen oder Prothesen, die ein Oberteil und einem gelenkig daran gelagertes Unterteil aufweisen. Bei Orthesen werden das Oberteil und das Unterteil an einer noch vorhandenen Gliedmaße festgelegt, beispielsweise durch Schalen, Gurte, Riemen, Manschetten oder andere Befestigungseinrichtungen. Über Orthesen können Bewegungen geführt, Verschwenkung um eine Gelenkachse begrenzt, Verschwenkbewegungen verhindert oder eine Ausrichtung von Gliedmaßen zueinander unterstützt oder festgelegt werden. Darüber hinaus können Orthesen mit Brems- oder Dämpferelementen versehen sein, um eine Verschwenkbewegung um die Gelenkachse zu dämpfen oder zu blockieren. Die Brems- oder Dämpfereinrichtungen können mit einer Steuerung versehen sein, sodass in Abhängigkeit von Sensordaten eine veränderte Dämpfung in Flexionsrichtung und/oder Extensionsrichtung bereitgestellt oder die Bewegung werden kann. Ebenfalls ist es bekannt, Kraftspeicher dem Oberteil bzw. dem Unterteil zuzuordnen, sodass eine Bewegungsunterstützung über eine Freigabe der gespeicherten Energie aus dem Kraftspeicher erfolgen kann.

Prothesen ersetzen eine nicht vorhandene oder nicht mehr vorhandene Gliedmaße und dienen zur Bereitstellung einer Funktionalität, die der Funktionalität der natürlichen Gliedmaße möglichst angenähert ist. Darüber hinaus dienen Prothesen dazu, ein möglichst natürliches Erscheinungsbild für den Prothesennutzer bereitzustellen. Ein Prothesenoberteil ist beispielsweise als ein Prothesenschaft oder als eine an einem Prothesenschaft befestigte Komponente ausgebildet, bei der der Prothesenschaft zur Festlegung an einer Gliedmaße oder einem Gliedmaßenstümpfen dient. Das Prothesengelenk, beispielsweise ein Prothesenkniegelenk, ein Prothesenknöchelgelenk oder ein Prothesen-Ellenbogengelenk, verbindet das Oberteil mit einem Unterteil, das wiederum weitere prothetische Komponenten aufweisen kann, beispielsweise ein Unterschenkelrohr, ein Prothesenfuß oder eine Prothesenhand.

Insbesondere bei Orthesenkniegelenken und Prothesenkniegelenken sind zwischen dem Oberteil und dem Unterteil Dämpfer, insbesondere hydraulische Dämpfer, oder Brems- bzw. Widerstandseinrichtungen angeordnet, die auf Grundlage von Sensordaten unterschiedliche Widerstände in einzelnen Gangsituationen bereitstellen oder eine Bewegung vollständig blockieren. Solche Widerstandseinrichtungen sind häufig als hydraulische Linearaktuatoren ausgebildet, die einen definierten Widerstand gegen eine Flexionsbewegung oder Extensionsbewegung bereitstellen. Der Widerstand wird durch eine Veränderung der Stellung von Ventilen verändert. Bei der Verringerung des Strömungsquerschnittes erhöht sich der entsprechende Widerstand gegen eine Bewegung. Durch Sperren einer Verbindungsleitung über ein Stellventil kann eine Bewegung blockiert werden. Solche Stellventile, die beispielsweise für die Standphasendämpfung eingesetzt werden, können mechatronisch über Servoventile oder mechanisch über ein Drosselventil eingestellt werden. Alternativ zu einem hydraulischen Linearaktuator können auch andere, beispielsweise elektromechanische Linearaktuatoren oder linear wirkende Gesperre eingesetzt werden.

Die Erhöhung eines Flexionswiderstandes bis hin zu einer Sperrung der Gelenkeinrichtung kann aus unterschiedlichen Gründen notwendig sein. Je nach Ausgestaltung der orthopädietechnischen Gelenkeinrichtung erfolgt eine Sperrung rein mechanisch, beispielsweise durch Einrasten eines Sperrelementes in einer Ausnehmung oder durch eine andere, formschlüssige oder reibschlüssige Blockierung des Oberteils relativ zu dem Unterteil. Alternativ kann die Erhöhung eines verstellbaren Widerstandes auf ein Niveau erfolgen, das eine weitere Bewegung praktisch unmöglich macht, beispielsweise durch Schließen eines Ventils, die Verstärkung eines Magnetfeldes zur Veränderung einer Viskosität einer magnetorheologische Flüssigkeit oder durch entsprechende Erhöhung eines elektromechanischen Widerstandes in einem Generatorprinzip eines Motors. Eine vollständige Sperrung der Gelenkeinrichtung in beiden Bewegungsrichtungen kann jedoch auch nachteilig sein, insbesondere wenn damit auch die Sperrung einer Extensionsbewegung verbunden ist.

Die EP 1 996 128 B1 betrifft eine Gelenkeinrichtung mit einem Oberteil und einem um eine Schwenkachse drehbar daran gelagerten Unterteil. Die Gelenkeinrichtung weist eine Spiralfeder auf, die um eine Verdrehachse parallel zu der Schwenkachse verspannbar gelagert ist. Die Spiralfeder ist zwischen zwei Anlaufflächen angeordnet und tritt bei Verspannung in Richtung auf die Anlaufflächen mit diesen in Kontakt.

Über einen Hebel kann die Spiralfeder verspannt oder entspannt werden, sodass die Reibung zwischen der Spiralfeder und den Anlaufflächen vergrößert, verkleinert oder aufgehoben wird.

Die DE 334 426 C betrifft ein Gelenk mit einer inneren Scheibe und einer koaxial dazu angeordneten äußeren Scheibe. An der inneren Scheibe sind Sperrzähne angeordnet, die bei einer Verdrehung der inneren Scheibe in eine erste Richtung um eine Schwenkachse in Sperrrasten der äußeren Scheibe eingreifen. Das Gelenk weist ein Drehteil mit daran angeordneten Stiften auf. Bei einer Verdrehung des Drehteils in die erste Richtung um die Schwenkachse treten die Stifte in Kontakt mit den Sperrzähnen und verschwenken diese um einen Lagerpunkt, sodass diese nicht mehr in die Sperrrasten der äußeren Scheibe eingreifen. In eine zweite Richtung, die der ersten Richtung entgegengerichtet ist, kann die innere Scheibe stets frei um die Schwenkachse verdreht werden.

Die DE 10 2018 217497 A1 betrifft eine Gelenkvorrichtung mit einem äußeren Kupplungsring und einem inneren Abstützring. In dem Kupplungsring sind mehrere zum Abstützring hin offen ausgebildete Aussparungen angeordnet, die ein entlang der Umfangsrichtung verjüngtes Ende aufweisen. In den Aussparungen sind Stifte angeordnet, die entlang der Umfangsrichtung in der Aussparung zum verjüngten Ende hin oder von dem verjüngten Ende weg bewegbar sind.

Die US 6 517 503 B1 betrifft eine Gelenkvorrichtung mit einer oberen Rasterplatte und einer unteren Rasterplatte. Die beiden Rasterplatten weisen an den zueinander ausgerichteten Seiten jeweils Rastzähne auf. Eine Tellerfeder hält die obere Rasterplatte in einer Freigabestellung, in der die obere Rasterplatte von der unteren Rasterplatte beabstandet ist. Mithilfe einer elektromagnetischen Spule kann die obere Rasterplatte angezogen werden, sodass sie mit der unteren Rasterplatte in Eingriff gebracht wird.

Die EP 2 502 607 B1 betrifft eine Gelenkvorrichtung mit einem inneren Ring und einem äußeren Ring. Der innere Ring weist Aussparungen auf, die zum äußeren Ring hin offen sind. In den Aussparungen sind Rollen angeordnet, die mit einer Feder in Richtung zu einem verjüngten Ende der Aussparung vorgespannt sind und so einen Reibschluss zwischen dem inneren Ring und dem äußeren Ring herstellen. An zwei Rädern sind Stifte angeordnet, die sich in jeweils eine Aussparung erstrecken. Durch ein Verschwenken der Räder können die Stifte gegen die Rollen gedrückt werden, sodass die Rollen aus dem verjüngten Ende bewegt werden und kein Reibschluss zwischen dem inneren Ring und dem äußeren Ring besteht.

Aufgabe der vorliegenden Erfindung ist es daher, eine orthopädietechnische Gelenkeinrichtung bereitzustellen, mit der eine erhöhte Sicherheit bei der Benutzung mit einfachen Mitteln bereitgestellt werden kann.

Erfindungsgemäß wird diese Aufgabe durch eine orthopädietechnische Gelenkeinrichtung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die orthopädietechnische Gelenkeinrichtung mit einem Oberteil und einem um eine Schwenkachse schwenkbar daran gelagerten Unterteil sowie einer Sperreinrichtung, die eine Verschwenkung des Oberteils relativ zum Unterteil sperrt sieht vor, dass die Sperreinrichtung in einer Schwenkrichtung nie sperrt und in der entgegengesetzten Schwenkrichtung schaltbar aus einer Freigabestellung in eine Sperrstellung bringbar ist. Mit einer solchen orthopädietechnischen Gelenkeinrichtung ist es möglich, eine Sperrung in eine Richtung in einer beliebigen Position des Oberteils relativ zu dem Unterteil zu ermöglichen, während die Bewegung in der Gegenrichtung immer möglich ist. Die Sperrung des Gelenkes erfolgt in einer Ausgestaltung als ein Kniegelenk in einer Orthese oder Prothese vorteilhafterweise in der Standphase, wobei die Sperrung in Flexionsrichtung während der Schwungphase oder zur Einleitung der Schwungphase aufgehoben wird. Auf diese Weise ergibt sich eine standphasenkontrollierte Sperrung der Flexion bei einer Schwungphasenfreischaltung. Die Sperreinrichtung ist mit einem Überlastschutz ausgestattet, der bewirkt, dass bei einer Überlastung der orthopädietechnischen Gelenkeinrichtung das Sperrelement durchrutscht und somit das Oberteil gegenüber dem Unterteil verschwenkt werden kann. Dazu kann beispielsweise das Sperrelement ab einer bestimmten Belastung auf einem Sperrbereich entlang gleiten, wozu das Material und die Oberflächenbeschaffenheit sowohl des Sperrelementes als auch des Sperrbereichs entsprechend gewählt werden.

Der Sperreinrichtung ist ein Betätigungselement zugeordnet, das die Sperreinrichtung in der Freigabestellung hält oder in die Freigabestellung bewegt. Dieses Betätigungselement sorgt dafür, dass die Sperreinrichtung entweder in der Grundeinstellung in der Freigabestellung befindlich ist oder aber nach Erhalt eines Steuerungssignals oder aufgrund einer bestimmten Belastung, einer entsprechenden Kraftleistung auf das Betätigungselement oder eines Steuerelementes oder bei einer bestimmten Bewegungssituation oder Belastungssituation in die Freigabestellung bewegt wird. Hält das Betätigungselement die Sperreinrichtung in der Freigabestellung, ist die grundsätzliche Einstellung die bewegliche Einstellung, sodass eine Verschwenkung in die erste Schwenkrichtung, beispielsweise in die Flexionsrichtung bei einem Kniegelenk, ebenso freigegeben ist wie in die entgegengesetzte Schwenkrichtung, also Extensionsrichtung. Erst bei der Detektion einer entsprechenden Belastungssituation oder Bewegungssituation wird die Sperreinrichtung aus der Freigabestellung in die Sperrstellung bewegt, sodass die Verschwenkung des Oberteils relativ zu dem Unterteil gesperrt wird. Das Betätigungselement kann die Sperreinrichtung auch in die Freigabestellung bewegen, wenn sich die Sperreinrichtung in der Sperrstellung befindet, beispielsweise wenn die Gelenkeinrichtung aufgrund einer bestimmten Belastungssituation gesperrt wurde und die Belastungssituation nicht mehr gegeben ist. Das Betätigungselement kann beispielsweise ein Hebel, ein Stift, ein Zugelement, ein Exzenter, ein Lenker oder eine andere Kraftübertragungseinrichtung sein.

In einer Ausgestaltung ist dem Betätigungselement ein Aktuator zugeordnet, der das Betätigungselement in der Freigabestellung hält oder in die Freigabestellung bewegt, wobei die Freigabestellung des Betätigungselementes die Freigabestellung der Sperreinrichtung ist. In der Freigabestellung des Betätigungselementes ist das Oberteil relativ zu dem Unterteil in derjenigen Schwenkrichtung beweglich, in der die Sperreinrichtung die orthopädietechnische Gelenkeinrichtung sperren kann. Der Aktuator kann beispielsweise als Magnetschalter, Motor, schaltbarer Federspeicher, Pneumatikelement, Hydraulikaktuator, Bowdenzug Solenoid und/oder ein anderes Element sein, das dem Betätigungselement Energie zuführt und es bewegt oder in der Freigabestellung hält.

Zusätzlich zu dem Aktuator kann auch ein mechanisches Schaltelement vorgesehen sein, um das Sperrelement manuell permanent in einem der Zustände zu halten, entweder in der gesperrten Stellung oder in der freigegebenen Stellung. In dem Fall einer elektronischen oder elektromechanischen Betätigung kann dies über manuell bedienbare Steuerelemente, Fernbedienungen oder Einstellungen in externen Geräten, beispielsweise einem Smartphone, erfolgen. Der permanent freigegebene Zustand ist insbesondere beim Anlegen der Orthese oder beim Fahrradfahren relevant. Für Aktivitäten, bei denen es auf eine zuverlässige Standsicherung ankommt, ist der permanent gesperrte Zustand vorteilhaft.

Die Sperreinrichtung weist zumindest ein Sperrelement auf, das verschwenkbar oder verschieblich an dem Oberteil gelagert und mit einem Sperrbereich an oder in dem Unterteil in Eingriff bringbar ist oder umgekehrt an dem Unterteil gelagert und mit einem Sperrbereich an oder in dem Oberteil in Eingriff bringbar ist. Auf diese Weise wird eine mechanische Kopplung zwischen dem Oberteil und dem Unterteil bewirkt und eine entsprechende Sperrung einer Verschwenkung in einer Schwenkrichtung ausgeführt. Der Sperrbereich und/oder die Lagerung der Sperrelemente oder des Sperrelementes sind so ausgebildet, dass in der entgegengesetzten Schwenkrichtung keine Sperrwirkung auftritt und eine Verschwenkung und Relativbewegung von Oberteil zu Unterteil stets möglich ist.

Das Sperrelement kann als Sperrbügel, Sperrwinkel, Sperrkeil, Bremsbelag, Sperrhaken oder Sperrkissen ausgebildet sein, der oder das in der Sperrstellung an dem Sperrbereich anliegt und dort entweder über Reibkräfte oder über Formschluss in Kontakt gehalten wird und eine Sperrung der Verschwenkbewegung in der einen Richtung bewirkt. Ein Sperrbügel kann beispielsweise exzentrisch zu einer verschwenkbaren Reibfläche eines Sperrbereichs gelagert sein und auf dieser entlang gleiten. Das Sperrelement steht in einer Ausgestaltung in einem reibschlüssigen Kontakt mit dem Sperrbereich. In der ersten Schwenkrichtung wird der Sperrbügel über die Reibkräfte angehoben und in der Freigabestellung gehalten, in der anderen Schwenkrichtung wird eine selbstverstärkende Blockierung der Gelenkeinrichtung bewirkt und das Oberteil relativ zu dem Unterteil verriegelt. Sperrkissen oder Sperrkeile, die verschwenkbar oder verformbar beispielsweise an dem Oberteil anliegen und auf dem Sperrbereich an einer Reibfläche entlang gleiten, verkeilen sich in der einen Schwenkrichtung und sperren das Gelenk und gleiten ohne Beeinträchtigung oder Blockierung der Verschwenkbewegung in der anderen Schwenkrichtung auf dem Sperrbereich oder der Reibfläche entlang. Durch Abheben dieser Sperrelemente von der Reibfläche kann die Bewegung in beide Richtungen freigegeben werden. Vorteilhafterweise sind die Sperrelemente so ausgestaltet, dass sie sich in einer Art Kettenreaktion gegenseitig anheben, sofern der Betätigungsmechanismus zur Freischaltung der Bewegung in beide Richtungen aktiviert wurde.

Das Sperrelement ist in einer Ausgestaltung elastisch in Richtung auf den Sperrbereich vorgespannt, sodass sichergestellt ist, dass grundsätzlich ein Reibkontakt oder ein physischer Kontakt zwischen dem Sperrelement und dem Sperrbereich vorhanden ist. Dadurch ist sichergestellt, dass das Sperrelement jederzeit bei einer entsprechenden Belastung und Bewegung bzw. einer entsprechenden Schaltung des Betätigungselementes oder des Aktuators eine Sperrstellung erreichbar ist, sodass ohne Zeitverzögerung eine sichere Sperrung des Gelenkes in der jeweiligen Schwenkrichtung erfolgt. Darüber hinaus können durch diese Vorspannelemente Formänderungen z.B. aufgrund von Temperatur, Feuchtigkeit oder Verschleiß ausgeglichen werden.

Die elastische Vorspannung des Sperrelementes erfolgt in einer Ausgestaltung durch ein Vorspannelement, das elastisch ausgebildet oder elastisch gelagert ist, wobei wahlweise die Steifigkeit des elastischen Vorspannelementes und/oder dessen Vorspannung einstellbar ist. Dadurch kann eine Anlagekraft an den Sperrbereich oder eine Sperrfläche eingestellt werden. Bei einer Kombination des Sperrelementes mit einem Betätigungselement oder einem Aktuator werden über das Vorspannelement diejenigen Betätigungskräfte definiert oder beeinflusst, die notwendig sind, um die Sperreinrichtung oder das Sperrelement in die Freigabestellung zu bewegen oder in der Freigabestellung zu halten. Dadurch kann eine Anpassung der orthopädietechnischen Gelenkeinrichtung an den jeweiligen Einsatzzweck oder ein Verschleißausgleich erfolgen.

**In** einer Ausgestaltung ist der Sperreinrichtung ein elastisches Element vorgelagert, dessen Steifigkeit und/oder Vorspannung einstellbar ist. Die Einstellung der Steifigkeit und/oder Vorspannung erfolgt in einer Ausgestaltung ebenfalls über ein verstellbares Auflager, beispielsweise einen verlagerbaren Stift oder einen verlagerbaren Bolzen, über den die effektive Länge des elastischen Elementes verändert und/oder die Vorspannung verändert wird. Bei der Verkürzung der effektiven Länge erhöht sich die Steifigkeit des elastischen Elementes, bei einer Verlängerung verringert sich die Steifigkeit. Auch die Vorspannung des elastischen Elements kann durch Verlagerung eines der Auflagerpunkte des elastischen Elements in dessen Wirkrichtung verstellt werden. Durch Verschiebung des Auflagerpunkts in zwei verschiedene Richtungen kann somit sowohl die Steifigkeit als auch die Vorspannung des elastischen Elementes beeinflusst werden.

Das Sperrelement kann an einem Kopplungselement gelagert sein, das starr oder elastisch mit dem Oberteil oder dem Unterteil verbunden ist. Die Außenkontur des Kopplungselementes muss nicht notwendigerweise rund sein. Dieses Kopplungselement ist in einer Ausgestaltung mit einer Überlastkupplung versehen. Der Überlastschutz kann auch durch ein Klemmelement verwirklicht werden, wodurch im Überlastfall ein paralleler Kraftschluss entsteht.

In einer Ausgestaltung ist die Sperreinrichtung über eine zentrale Lagerung geführt bzw. an einer zentralen Lagerung gelagert. Die zentrale Lagerung oder die zentrale Lagerstelle kann mit dem Oberteil und mit dem Unterteil in einem direkten Kontakt stehen, sodass beispielsweise über ein Wälzlager oder ein Gleitlager ein Zapfen eines ersten Teils mit einer Buchse oder Hülse eines zweiten Teils drehbar gelagert ist. Auch die Sperreinrichtung kann an der zentralen Lagerung oder dem zentralen Lager gelagert sein und mit dem Oberteil und Unterteil in einem direkten Kontakt stehen. Insbesondere ist die Sperreinrichtung zwischen dem Oberteil und dem Unterteil zwischen zwei hülsenartigen Komponenten angeordnet, wobei die Anordnung insbesondere so gewählt ist, dass um einen Zapfen eines ersten Teils die zentrale Lagerung angeordnet ist, um die herum eine Hülse des zweiten Teils anliegt oder entlang gleitet. Auf der Außenseite der Hülse des zweiten Teils liegt oder greift die Sperreinrichtung an, die auf der Innenseite eine Reibfläche oder eine Anlagefläche oder korrespondierenden Kontaktfläche für die Sperreinrichtung an dem ersten Teil anliegt.

Die zum Umschalten in den freien Zustand erforderliche Kraft an dem Betätigungselement ist vorteilhafterweise gering ist, sofern keine Last entgegen der Sperrrichtung anliegt. Liegt hingegen eine Last entgegen der Sperrrichtung an, erhöht sich die zum Umschalten in den freien Zustand erforderliche Kraft an dem Betätigungselement, insbesondere erhöht sich die erforderliche Kraft mit der Zunahme der Last.

Die durch den Aktuator aufgebrachte Kraft an dem Betätigungselement kann begrenzt sein oder begrenzbar, insbesondere einstellbar sein. Dadurch kann gewährleistet werden, dass keine ungewollte Umschaltung durch den Aktuator bei einem oder ab einem bestimmten Lastniveau erfolgt. Der Aktuator ist aufgrund der Leistungsbegrenzung nicht in der Lage, bei einer kritischen Last in die Freigabestellung zu schalten, was eine erhöhte Sicherheit gewährleistet.

Der Umstand, dass die von einem Aktuator aufzubringende Kraft an dem Betätigungselement einstellbar und begrenzbar ist, lässt sich zur Steuerung oder Anpassung der orthopädietechnischen Gelenkeinrichtung verwenden. Über die eingestellte Aktuatorkraft, die auch durch die Auswahl des Aktuators erfolgen kann, wird eine Sicherheit gegen unbeabsichtigtes Umschalten in den freien Zustand bei Belastung entgegen der Sperrrichtung erreicht. Der Belastungszustand der Sperreinrichtung wird beispielsweise anhand des Zustands oder der Energieaufnahme des Aktuators erfasst oder abgeschätzt. Diese Art der Steuerung ist unabhängig von dem konkreten Aufbau der orthopädietechnischen Gelenkeinrichtung.

**In** einer Ausgestaltung weist die Sperreinrichtung einen Außenring und einen Innenring auf, zwischen denen das zumindest eine Sperrelement angeordnet ist. Das Sperrelement oder die Sperrelemente sind verlagerbar zwischen dem Innenring und dem Außenring gelagert und können hinsichtlich der Position und/oder Orientierung relativ zu dem Außenring bzw. Innenring eingestellt werden.

**In** einer Ausgestaltung ist der Außenring elastisch ausgebildet oder geschlitzt ausgebildet, wobei der Schlitz in Radialrichtung verläuft und den Umfang des Außenringes durchbricht. Dadurch ist es möglich, den Innenumfang des Außenringes zu verändern. Insbesondere wenn der Außenring aus einem elastischen Material ausgebildet ist, besteht die Möglichkeit, durch eine Vorspanneinrichtung oder eine Einstelleinrichtung den Umfang und/oder die Vorspannung zu verändern. Wird die Vorspanneinrichtung gespannt, kann der Innendurchmesser des Außenringes verringert bzw. die Vorspannung des Außenringes vergrößert werden. Bei einer Entspannung des Außenringes wird der Innenumfang vergrößert und die Vorspannung verringert. Durch die Veränderung des Innenumfanges und/oder der Vorspannung ist es möglich, bei einer entsprechend elastischen Ausgestaltung des Außenringes eine gewisse Elastizität des Oberteils relativ zu dem Unterteil eine Verdrehung auch in Sperrrichtung, bei der sich die Sperrelemente in der Sperrstellung befinden, zu erreichen. Diese Drehelastizität ist einstellbar. Durch ein entsprechendes Materialwahl, Lagerung und Formgebung des Außenringes ist es möglich, auch bei einem geschlossenen Querschnitt eine radiale Aufdehnung zumindest bereichsweise zu erreichen.

Die Elastizität des Außenringes kann durch die Wahl des Materials des Außenringes und/oder die Wandstärke des Außenringes eingestellt werden. Je nach Wahl des Materials und/oder der Wandstärke wird eine vergrößerte oder verringerte Elastizität des Außenringes erreicht. Bei einem vorgegebenen Außenumfang bei einem geschlossenen Außenring wird die Elastizität in einer Ausführungsform durch die Wahl des Innendurchmessers des Außenringes eingestellt. Alternativ oder ergänzend wird die Elastizität des Außenringes über die axiale Breite eingestellt. Je breiter der Außenring ist, desto steifer ist der Außenring bzw. desto geringer ist die Elastizität.

**In** einer Ausgestaltung ist der Außenring, insbesondere wenn er geschlitzt ausgebildet und mit einem offenen Querschnitt versehen ist, verdrehgesichert in einem Gehäuse oder einer anderen Komponente des Oberteils bzw. Unterteils eingebettet oder festgelegt. Dadurch wird es einfach möglich, eine angepasste Veränderung des Innendurchmessers bzw. der Vorspannung über eine Einstelleinrichtung oder Vorspanneinrichtung zu erreichen. Bei der geschlitzten Ausgestaltung erfolgt die verdreggesicherte Festlegung bevorzugt nur an einem Ende, das gegenüberliegende Ende des Außenringes ist zumindest in Richtung auf das gegenüberliegende Ende des Außenringes verlagerbar, da andererseits keine Veränderung des Innendurchmesser erreicht werden kann. Die Vorspanneinrichtung ist beispielsweise als Spannschraube oder Spannelement ausgebildet, die manuell oder motorisch betrieben werden kann.

Bei einer Bewegung des Innenringes gegenüber dem Außenring entgegen der Sperrrichtung verkanten sich die Sperrelemente oder Sperrkörper, sodass sie sich zwischen dem Innenring und dem Außenring verspreizen und einklemmen. Die Sperrelemente versuchen den Außenring aufzudehnen, wodurch eine Zugspannung innerhalb des Außenringes entsteht. Die dadurch erzeugte elastische Verformung des Außenringes bewirkt ein elastisches Verhalten der Sperrmechanik entgegen der Sperrrichtung. Trotz der in Sperrstellung befindlichen Sperrelemente wird durch die elastische Verformung des Außenringes bei einer weiteren Belastung in Sperrrichtung eine geringfügige, elastische Bewegbarkeit des Oberteils relativ zu dem Unterteil zugelassen. Dadurch werden abrupte Sperreinstellungen vermieden, so dass beispielsweise bei plötzlicher Aktivierung der Sperre eine gewisse elastische Nachgiebigkeit erreicht wird, um das Material der orthopädietechnischen Einrichtung und den Körper des Patienten zu schonen. Durch eine passende Wahl des Materials und des Querschnittes des Außenringes, also die Wandstärke und die Breite des Außenringes, kann die Elastizität beeinflusst bzw. eingestellt werden, um ein für die jeweilige Anwendung günstiges Verhalten zu erzielen. Auch andere Arten einer Anpassung der Elastizität des Außenringes über den Querschnitt sind möglich. So können beispielsweise durch eine Kombination verschiedener Materialien oder eine besondere Formgebung durch Rillen, Kerben oder dergleichen in gleichmäßiger oder ungleichmäßiger Verteilung über den Umfang herum die gewünschten Elastizitäten erreicht werden. Insbesondere ist es für den Einsatz in künstlichen Kniegelenken vorteilhaft, eine Elastizität entgegen der Sperreinrichtung durch entsprechende Formgebung und Ausgestaltungen des Außenringes auf einen Bereich zwischen 2 Nm/° und 20 Nm/° (pro Winkelgrad Verschwenkung) einzustellen, um eine Energierekuperation, insbesondere während der Standphase, zu ermöglichen.

Die Elastizität der Sperreinrichtung kann auch durch die Weite der Geometrie der Sperrelemente verändert werden. Die Elastizität der Sperreinrichtung ergibt sich durch die Drehung des Außenringes relativ zu dem Innenring bei einem gegebenen Moment, das an dem Außenring oder Innenring angebracht wird. Weiterhin ergibt sich eine Verdrehung des Außenringes durch die Verdrehung der Sperrelemente, die mit der Ausdehnung des Außenringes zusammenhängt. Das Ausmaß der Verdrehung der Sperrelemente ist von der Spaltweite zwischen dem Innenring und dem Außenring abhängig, die durch die Ausdehnung des Außenringes bestimmt wird. Da das Verhältnis zwischen Verdrehung der Sperrelemente und der Spaltweite von der Geometrie der Sperrelemente abhängig ist, können gezielte Elastizitäten der Sperreinrichtung über die Anpassung der Konturen der Sperrelemente verwirklicht werden.

Die orthopädietechnische Gelenkeinrichtung mit einem Oberteil und einem Unterteil, das über zumindest ein Gelenk verschwenkbar um eine Schwenkachse an dem Oberteil angeordnet ist, mit einem ersten Aktuator, der zwischen dem Oberteil und dem Unterteil angeordnet ist und ein erstes Einstellelement aufweist, über das der Bewegungswiderstand des ersten Aktuators gegen eine Verschwenkung des Unterteils relativ zu dem Oberteil um die Schwenkachse einstellbar ist, und mit zumindest einem zweiten Aktuator, der zwischen dem Oberteil und dem Unterteil angeordnet ist und zumindest ein zweites Einstellelement aufweist, über das der Bewegungswiderstand des zweiten Aktuators gegen eine Verschwenkung des Unterteils relativ zu dem Oberteil um die Schwenkachse einstellbar ist, mit zumindest einem Sensor und mit einer Steuereinheit, die mit dem zumindest einen Sensor zur Übermittlung von Sensordaten verbunden ist und das erste Einstellelement und das zweite Einstellelement in Abhängigkeit von Sensordaten steuert, sieht in einer Ausgestaltung vor, dass die Steuereinheit das erste Einstellelement und das zweite Einstellelement synchronisiert steuert bzw. eingerichtet ist, die Einstellelemente synchron zu steuern. Bei einer synchronisierten Steuerung passt die Steuereinheit die Steuerung der Einstellelemente zeitlich aneinander an oder stimmt sie aufeinander ab, sodass insbesondere synchronisiertes Freigeben und Sperren der Verschwenkung des Unterteils relativ zu dem Oberteil ermöglicht wird. Durch eine derartige synchronisierte Steuerung werden Überlastungen infolge von zu spät freigegebener oder zu früh gesperrter Verschwenkung verhindert. Darüber hinaus ergibt sich der Vorteil, dass keine aufwändigen Anpassungen und Einstellungen der Widerstandseinrichtungen durch einen Orthopädietechniker im Rahmen eines Einstellprozesses erforderlich ist. Weiterhin werden durch die Verwendung einer einzigen zentralen Steuereinheit zur Steuerung mehrerer Einstellelemente Redundanzen in der Sensorik und Hardware vermieden, woraus sich geringere Herstellungskosten, geringeres Gewicht und eine erhöhte Laufzeit der orthopädietechnischen Gelenkeinrichtung ergeben. Gleichzeitig wird dadurch eine kompaktere Bauweise der orthopädietechnischen Gelenkeinrichtung ermöglicht. Grundsätzlich können von einer Steuereinheit auch mehr als zwei Einstellelemente synchronisiert gesteuert werden.

Vorteilhafterweise sind die Aktuatoren motorische Antriebe, Federspeicher, Freigabe-und/oder Verriegelungseinrichtungen und/oder hydraulische und/oder pneumatische Widerstandseinrichtungen, insbesondere Rotationshydrauliken. Jeder Aktuator kann an einem Gelenk angeordnet sein oder dieses selbst ausbilden. Über das Einstellelement ist der Bewegungswiderstand des jeweiligen Aktuators gegen eine Verschwenkung des Unterteils relativ zu dem Oberteil einstellbar. Bei einer hydraulischen Widerstandseinrichtung kann das Einstellelement beispielsweise ein Steuerventil sein, über das der Widerstand der Widerstandseinrichtung verstellbar ist. Das Ventil kann insbesondere elektrisch oder elektronisch geschaltet werden. Ein Einstellelement kann sowohl mechanisch als auch motorisch oder elektronisch eine Veränderung des Widerstands des Aktuators herbeiführen. Wenn der Aktuator als elektromotorischer Antrieb ausgeführt ist, sind beispielsweise die am Kommutator anliegenden Bürsten oder die Spulen des Rotors als erfindungsgemäßes Einstellelement anzusehen.

Der zumindest eine Sensor kann als Kraftsensor, Winkelsensor, Wegsensor, Momentsensor, Beschleunigungssensor, Geschwindigkeitssensor oder Lagesensor ausgebildet sein. Dieser Sensor ist eingerichtet, wenigstens einen Messwert zu erfassen und an die Steuereinheit zu übermitteln. In Abhängigkeit von den Sensordaten kann die orthopädietechnische Gelenkeinrichtung gesteuert werden, um an den Nutzer oder an die jeweilige Bewegung oder Bewegungssituation angepasste Einstellungen vornehmen zu können.

Eine Weiterbildung sieht vor, dass der erste Aktuator ein erstes Gelenk aufweist, das eine erste Gelenkachse aufweist, die koaxial zu der Schwenkachse ist, und dass der zweite Aktuator ein zweites Gelenk aufweist, das eine zweite Gelenkachse aufweist, die ebenfalls koaxial zur Schwenkachse ist. Dadurch, dass die Aktuatoren separate Gelenke ausbilden, wird die Stabilität und Tragfähigkeit der orthopädietechnischen Gelenkeinrichtung verbessert. Die Koaxialität der Gelenkachsen mit der Schwenkachse bewirkt, dass keine ungewünschten Momente und/oder Lasten bei einer Verschwenkung des Unterteils relativ zu dem Oberteil entstehen. Bevorzugt ist die Schwenkachse koaxial zu der natürlichen Gelenkachse oder einer Kompromissgelenkachse, um ein möglichst natürliches Bewegungsverhalten zu erreichen.

Vorzugsweise ist der erste Aktuator lateral angeordnet und der zweite Aktuator medial angeordnet. Die bilaterale Anordnung der Aktuatoren führt zu einer weiteren Erhöhung der mechanischen Tragfähigkeit der orthopädietechnischen Gelenkeinrichtung. Insbesondere bei der bilateralen Anordnung der Aktuatoren beispielsweise bei einer Knieorthese ist eine synchronisierte Steuerung der Einstellelemente erforderlich, um ungewollte Knieflexionen und Knieextensionen zu vermeiden. Aus der bilateralen Anordnung der Aktuatoren ergibt sich zudem eine Verringerung von Torsionslasten, die bei einer monolateralen Anordnung beispielsweise durch die einseitige Dämpfung der Verschwenkung des Unterteils relativ zu dem Oberteil entstehen können.

Bevorzugt basieren der erste Aktuator und der zweite Aktuator auf unterschiedlichen mechanischen Wirkprinzipien. Besonders bevorzugt ist der erste Aktuator als ein motorischer Antrieb oder eine hydraulische und/oder pneumatische Widerstandseinrichtung, insbesondere eine Rotationshydraulik, und der zweite Aktuator als eine Freigabe- und Verriegelungseinrichtung ausgebildet. Beispielsweise ist es von Vorteil, wenn ein motorischer Antrieb oder eine hydraulische und/oder pneumatische Widerstandseinrichtung, insbesondere eine Rotationshydraulik, lateral angeordnet ist und eine Freigabe- und Verriegelungseinrichtung medial. Freigabe- und Verriegelungseinrichtungen, insbesondere mechanische Freigabe- und Verriegelungseinrichtungen, sind einfach und platzsparend aufgebaut und ermöglichen, dass eine dauerhafte Verriegelung bereitgestellt wird, ohne dass Energie verbraucht wird. Darüber hinaus wird dadurch verhindert, dass während längere Standphasen aufgrund von Leckagen das Gelenk einbeugt, was bei hydraulischen oder pneumatischen Widerstandseinrichtungen der Fall sein kann. In diesem Fall kann durch Verriegelung einer medial angeordneten Freigabe- und Verriegelungseinrichtung der sichere Stand des Benutzers gewährleistet werden. Bei einem elektromechanischen Aktuator kann in dieser Situation Energie gespart werden, da durch die parallel wirkende Verriegelungseinrichtung eine aktive Sicherung des Standes nicht erforderlich ist.

Eine Weiterbildung sieht vor, dass mehrere Aktuatoren medial und/oder lateral angeordnet sind. Auf diese Weise ist es möglich, die Anzahl der Aktuatoren an das Körpergewicht des Benutzers der orthopädietechnischen Gelenkeinrichtung anzupassen. Bevorzugt wird dabei ein modulares System verwendet, sodass eine schnelle und kostengünstige Anpassung der orthopädietechnischen Gelenkeinrichtung an das Körpergewicht des Benutzers erfolgen kann. Es kann auch von Vorteil sein, Aktuatoren ausschließlich lateral anzuordnen, um im medialen Bereich mehr Bewegungsfreiraum zu erzielen.

Vorzugsweise bildet die orthopädietechnische Gelenkeinrichtung ein künstliches Kniegelenk, Ellbogengelenk und/oder Knöchelgelenk aus. Die orthopädietechnische Gelenkeinrichtung kann auch mehrere Gelenke ausbilden, insbesondere ein künstliches Kniegelenk und ein künstliches Knöchelgelenk. Auch die Ausbildung mehrerer Gelenke der gleichen Art ist möglich, wenn beispielsweise mehr als zwei an zwei Extremitäten ein gleichartiges Gelenk ersetzt oder unterstützt werden soll.

Bevorzugt werden alle Einstellelemente durch eine einzige Steuereinheit gesteuert. Dadurch werden Redundanzen vermieden und die Steuereinheit kann die Steuerung aller Einstellelemente aneinander anpassen, sodass ein natürliches Bewegungsverhalten und eine präzisere, belastungsoptimierte Steuerung der Einstellelemente erzielt werden kann. Alternativ dazu kann eine Steuerung der Einstellelemente mit mehreren Steuereinheiten erfolgen. Bei einer orthopädietechnischen Gelenkeinrichtung, die mehrere Gelenke ausbildet, kann es beispielsweise von Vorteil sein, wenn die Einstellelemente eines jeden Gelenkes durch eine dem Gelenk zugehörige Steuereinheit gesteuert werden.

Vorteilhafterweise werden die Einstellelemente über Steuerleitungen oder ein BusSystem gesteuert. Besonders wenn eine große Anzahl an Einrichtungen durch das Steuergerät angesteuert werden, wird durch die Verwendung eines Bus-Systems die Anzahl der benötigten Datenleitungen deutlich reduziert.

Eine Weiterbildung sieht vor, dass die Steuereinheit mehrere Einstellelemente mit dem gleichen Steuersignal steuert. Insbesondere wenn die den jeweiligen Einstellelementen zugehörigen Aktuatoren auf dem gleichen mechanischen Wirkprinzip beruhen, bietet dies den Vorteil, dass die Aktuatoren der gleichen Belastung ausgesetzt werden und ein homogenes Reaktionsverhalten der Aktuatoren erreicht werden kann.

Durch lokale Verarbeitung eines einheitlichen Steuersignals auf Aktuatorebene kann auch bei unterschiedlichen Aktuator-Technologien ein synchronisiertes Verhalten erreicht werden.

Nachfolgend werden Ausführungsbeispiele anhand der Figuren näher erläutert. Gleiche Bezugszeichen bezeichnen gleiche Komponenten. Es zeigen:
- Figuren 1a bis 1c -: eine erste Ausgestaltung der Gelenkeinrichtung mit verschwenkbaren Sperrkeilen;
- Figur 1d -: eine abstrahierte Darstellung des Lagerkonzepts;
- Figur 2 -: eine Variante mit einem vorgelagerten, verstellbaren elastischen Element ;
- Figur 3 -: eine Variante mit einem elastischen Unterteil;
- Figur 4 -: eine Variante mit integrierter Elastizität;
- Figur 5 -: eine Variante mit einem Klemmring;
- Figur 6 -: eine Variante mit Sperrkeilen;
- Figur 7 -: eine Variante mit Sperrbügeln;
- Figur 8 -: eine Variante mit Bremsbelägen;
- Figur 9 -: Anwendungen der Gelenkeinrichtung;
- Figuren 10 bis 13 -: Varianten der orthopädietechnischen Gelenkeinrichtung im angelegten Zustand an einer rechten unteren Extremität in der Vorderansicht;
- Figur 14 -: eine Variante der orthopädietechnischen Gelenkeinrichtung in einem prothetischen Kniegelenk;
- Figur 15 -: eine Variante der Gelenkeinrichtung in Gestalt eines Kniegelenkes;
- Figur 16 -: einer Darstellung der Figur 15 ohne Abdeckung;
- Figur 17 -: eine Detailansicht des Oberteils gemäß Figur 16;
- Figur 18 -: eine Schnittdarstellung durch die Sperreinrichtung;
- Figur 19 -: eine schematische Darstellung der Sperreinrichtung mit Detaildarstellung;
- Figur 20 -: eine Darstellung mit Sperrelementfixierung am Innenring;
- Figur 21 -: eine Explosionsdarstellung der Figur 20;
- Figur 22 -: eine schematische Darstellung der Betätigung der Ausführungsform gemäß Figur 20;
- Figur 23 -: eine Detaildarstellung der Lagerung eines Sperrelementes;
- Figur 24 -: eine Ausführungsform mit am Innenring gelagerten Sperrelementen;
- Figur 25 -: eine Schnittdarstellung gemäß Figur 24 mit Vorspannelementen;
- Figur 26 -: eine schematische Darstellung der Betätigung der Ausführungsform gemäß Figur 25;
- Figuren 27 bis 29 -: Lagerkonzepte;
- Figur 30 -: eine Variante mit einem geschlitzten Außenring; sowie
- Figur 31 -: eine Detaildarstellung eines Sperrelementes.

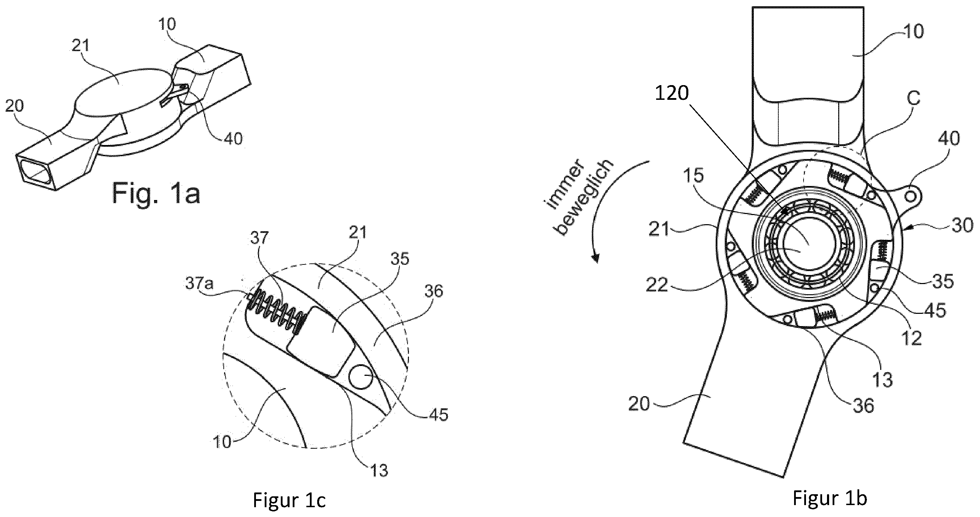

In der Figur 1a ist in einer perspektivischen Gesamtansicht eine orthopädietechnische Gelenkeinrichtung mit einem Oberteil 10 und einem Unterteil 20 dargestellt, die verschwenkbar aneinander gelagert sind. In der dargestellten Ausführungsform ist die orthopädietechnische Gelenkeinrichtung als Orthesengelenk, insbesondere als Orthesenkniegelenk dargestellt. Das Oberteil 10 und das Unterteil 20 weisen jeweils Aufnahmen für Orthesenschienen auf, die sich entlang einer Gliedmaße erstrecken und an der Gliedmaße mit entsprechenden Befestigungseinrichtungen wie Schalen, Gurten oder Schnallen festgelegt werden. Die orthopädietechnische Gelenkeinrichtung kann auch als Knöchelgelenk, Ellenbogengelenk oder ein anderes Gelenk ausgebildet sein. Grundsätzlich ist es auch möglich und vorgesehen, statt einer Orthese eine Prothese mit einer solchen Gelenkeinrichtung auszustatten. Die Ausführungen für Orthesen gelten entsprechend auch für Prothesen.

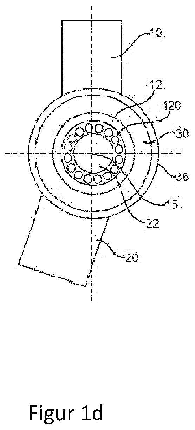

Die orthopädietechnische Gelenkeinrichtung gemäß der Figur 1 sieht in der dargestellten Ausführungsform an dem Oberteil 10 eine Lageraufnahme vor, in der das Unterteil 20 verschwenkbar gelagert ist. Das Unterteil weist ein Gehäuse 21 auf, in dem neben einem Lager auch eine Sperreinrichtung angeordnet ist, die später näher erläutert wird. Der Sperreinrichtung ist ein Betätigungselement 40 zugeordnet, das aus dem Gehäuse 21 herausragt und entweder von Hand oder motorisch bewegt werden kann. Die Funktionsweise der Sperreinrichtung wird nachfolgend erläutert.

In der Figur 1b ist in einer teiltransparenten Darstellung die orthopädietechnische Gelenkeinrichtung gemäß Figur 1a in Draufsicht gezeigt. Das Unterteil 20 mit dem Gehäuse 21 weist eine zylindrische Innenwand auf. Ebenso ist ein Zapfen 22 in dem Gehäuse 21 ausgebildet, der sich senkrecht zu der Blattebene erstreckt und als Aufnahme für ein zentrales Lager 120, in der dargestellten Ausführungsform ein Wälzlager dient. Dieses Wälzlager 120 als zentrales Lager ist in einer Lageraufnahme 12 des Oberteils 10 angeordnet. Alternativ zu der Lagerung in Gestalt eines Wälzlagers kann die Lagerung auch über ein Gleitlager oder über eine andere Lagertechnologie verwirklicht werden. Um die Lageraufnahme 12 herum sind Anlaufschrägen 13 ausgebildet, die sich in Richtung auf die zylindrische Innenwand des Gehäuses 21 erstrecken. Zwischen den Anlaufschrägen 13 und der Innenwand des Gehäuses 21 sind keilförmige Freiräume ausgebildet, in denen jeweils ein Sperrelement 35 als Teil der Sperreinrichtung 30 angeordnet sind. Die Sperrelemente 35, in dem dargestellten Ausführungsbeispiel fünf Sperrelemente 35, sind als Sperrkeile ausgebildet und stehen in Reibkontakt mit der Innenseite des Gehäuses 21. Zum Ausgleich von Fertigungstoleranzen können die Sperrkeile, wie in der Figur 1c dargestellt, bombiert ausgeführt werden. Um die lokalen Spannungen im Material zu minimieren, sind gleichmäßig an den Anlaufschrägen 13 bzw. dem Sperrbereich 36 anliegende Sperrkeile vorteilhaft. An der Innenseite des Gehäuses 21 wird dadurch ein Sperrbereich 36 ausgebildet, wenn die Sperrelemente 35 mit dem jeweiligen Flächenabschnitt der Innenwand des Gehäuses 21 in Eingriff treten. Wird das Oberteil 10 in der dargestellten Position gehalten und das Unterteil 20 entgegen dem Uhrzeigersinn verschwenkt, verdreht sich das Unterteil 20 gegenüber den Anlaufschrägen 13, die an dem Oberteil 10 ausgebildet oder angeordnet sind. Durch den Reibkontakt der Sperrkeile 35 mit der Innenwand des Gehäuses 21 werden die Sperrkeile 35 mitgenommen, sodass sie sich in einen sich vergrößernden Freiraum hinein bewegen. Dadurch geht der Reibkontakt zwischen den Sperrkeilen 35 und der Innenwand des Gehäuses 21 verloren oder reduziert sich ausreichend, sodass eine Bewegung des Unterteils 20 gegenüber dem Oberteil 10 entgegen dem Uhrzeigersinn möglich ist. Erfolgt eine entgegengesetzt gerichtete Bewegung des Unterteils 20 in Uhrzeigersinn um die Schwenkachse 15 herum, verkeilen sich die Sperrkeile 35 in dem sich verjüngenden Freiraum, sodass eine Sperrung der Relativbewegung zwischen Oberteil 10 und Unterteil 20 bewirkt wird. Eine Bewegung des Unterteils 20 entgegen dem Uhrzeigersinn ist immer möglich, eine Bewegung des Unterteils 20 im Uhrzeigersinn wird immer gesperrt. Je nach Orientierung der Anlaufschrägen 13 kann die Drehrichtung, in der stets eine Sperrung erfolgt und in der stets eine Bewegung möglich ist, eingestellt werden. Bei einer spiegelbildlichen Ausführung würde die Sperre in der entgegengesetzten Richtung wirken.

In einer Variante kann auf die zentrale Lagerung, die in dem Ausführungsbeispiel der Figur 1 durch ein Wälzlager verwirklicht wird, auch verzichtet werden, sofern sichergestellt ist, dass eine ausreichende Lagerung und Führung durch die Außenränder der Anlaufschrägen in dem Oberteil 10 gegenüber der zylindrischen Innenfläche des Gehäuses 21 an dem Unterteil 20 gewährleistet ist. Dies stellt hohe Anforderungen an die Fertigungsgenauigkeit der Komponenten.

Um zu ermöglichen, dass die orthopädietechnische Gelenkeinrichtung in beiden Schwenkrichtungen eine Schwenkbewegung des Oberteils 10 relativ zu dem Unterteil 20 ermöglicht, ist den Sperrelementen 35 über das Betätigungselement 40 jeweils ein Entriegelungsstift 45 zugeordnet. Das Betätigungselement 40 ist als Scheibe ausgebildet, die innerhalb des Gehäuses 21 gelagert ist und ebenfalls um die Schwenkachse 15 herum bewegt werden kann. Dazu ist in dem Gehäuse 21 ein Schlitz ausgebildet, der in der Figur 1a gezeigt ist. Wird das Betätigungselement 40 entgegen dem Uhrzeigersinn relativ zu dem Oberteil 10 bewegt, verdrehen sich die Entriegelungsstifte 45 relativ zu dem Oberteil 10 um die Schwenkachse 15 und bewegen die Sperrelemente 35 in Richtung auf die rückwärtige Wand der sich in diese Richtung öffnenden und vergrößernden Freiräume. Dadurch bringen sie die Sperrelemente 35 außer Eingriff mit den Sperrbereich 36 bzw. den Anlageflächen der Sperrelemente 35 an der Innenseite des Gehäuses 21, sodass eine Freigabe der Gelenkeinrichtung in beiden Bewegungsrichtungen erfolgt.

In der Figur 1c ist eine Detailansicht C der Figur 1b dargestellt. Das Sperrelement 35 in Gestalt eines Sperrkeils ist zwischen der Anlaufschräge 13 des Oberteils 10 und dem Sperrbereich 36 an der Innenseite des Gehäuses 21 des Unterteils 20 angeordnet und liegt an beiden Flächen an. Um die Anlage zu gewährleisten, ist dem Sperrelement 35 ein Vorspannelement 37 zugeordnet, das in der dargestellten Ausführungsform als eine Feder ausgebildet ist. Über das Vorspannelement 37, das um eine Führung 37a für das Sperrelement 35 herum angeordnet ist, können Toleranzen und Verschleiß der Opponenten ausgeglichen werden. In dem dargestellten Ausführungsbeispiel befindet sich der Entriegelungsstift 45 nicht in Kontakt mit dem Sperrelement 35, sodass eine Verlagerung des Unterteils 20 relativ zu dem Oberteil 10 nur dann möglich ist, wenn das Unterteil 20 bei feststehendem Oberteil 10 entgegen dem Uhrzeigersinn verdreht wird. Die Außenseite des Sperrelementes 35 liegt dann an dem Sperrbereich 36 an. Die vorhandenen Reibkräfte bewirken eine Verlagerung des Sperrelementes 35 entgegen der Vorspannung des Vorspannelementes 37 in Richtung auf die Rückwand des sich in diese Richtung öffnenden Freiraumes.

Dadurch wird eine Sperrwirkung aufgehoben und das Unterteil 20 kann entgegen dem Uhrzeigersinn relativ zu dem Oberteil 10 verschwenkt werden. Eine entgegengesetzte Bewegung des Unterteils 20 bewirkt, dass das Sperrelement 35 durch das Vorspannelement 37 gegen den Sperrbereich 36 gedrückt wird und sich zwischen der Anlaufschrägen 13 und der Sperrfläche 36 verkeilt. Eine weitergehende Bewegung des Unterteils 20 im Uhrzeigersinn wird verhindert. Werden größere Kräfte aufgebracht, verstärkt sich der Sperreffekt. Um diese Sperre zu lösen, werden die Entriegelungsstifte 45 entgegen dem Uhrzeigersinn in Richtung auf das Sperrelement 35 bewegt, wodurch das Sperrelement 35 außer Eingriff mit dem Sperrbereich 36 gelangt und dadurch eine freie Beweglichkeit sowohl im Uhrzeigersinn als auch entgegen dem Uhrzeigersinn möglich ist. Das Sperrelement wird durch ein optionales, zusätzliches Führungselement 37a geführt, um ein unbeabsichtigtes Verkanten in dem freigeschalteten Zustand zu verhindern.

Das Betätigungselement 40 kann permanent in einer Freigabestellung gehalten werden, sodass stets eine freie Beweglichkeit des Oberteils 10 relativ zu dem Unterteil 20 möglich ist. Eine solche freie Beweglichkeit kann beispielsweise gewünscht sein, wenn sich eine Person in einer sitzenden Stellung befindet. Das Betätigungselement 40 kann in der Freigabestellung fixiert werden. Wird die Fixierung aufgehoben, beispielsweise wenn sich die Person erheben will, ist eine Extensionsbewegung stets möglich. Eine Flexionsbewegung wird automatisch gesperrt und nur dann aufgehoben, wenn die Sperreinrichtung 30 mit dem Betätigungselement 40 und den Entriegelungsstiften 45 entsprechend verdreht werden. Über einen manuellen Schalter kann sowohl eine permanente Freischaltung als auch eine permanente Aktivierung der Flexionssperre realisiert werden. Alternativ oder ergänzend kann die Freischaltung auch automatisch, beispielsweise belastungsabhängig, insbesondere bei Überschreiten eines vorgegebenen Extensionsmoment oder elektronisch über einen Betätigungsaktuator abhängig von Sensorsignalen oder Anwenderbefehlen, z.B. über Bedienelemente, eine Fernbedienung oder Smartphone App erfolgen.

Figur 1d zeigt eine abstrahierte Darstellung der Figur 1, um das Konzept der konzentrischen Anordnung von zentralem Lager 120 und Sperreinrichtung 30 im Detail zu erläutern. Durch diese Anordnung ist sichergestellt, dass die nicht dargestellten Sperrelemente der Sperreinrichtung 30 frei von seitlichen Belastungen und Verkippungen sind, da die Funktionen "Lagerung" und "schaltbare Sperreinrichtung" von verschiedenen Bauteilen übernommen werden. Damit kann eine sichere Funktion und ein zuverlässiges Freischalten durch den nicht dargestellten Betätigungsmechanismus 40 sichergestellt wird. Durch das schematisch als Wälzlager dargestellte zentrale Lager 120 werden die dem Oberteil 10 zugehörige Lageraufnahme 12 und der dem Unterteil 20 zugehörige Zapfen 22 zueinander drehbar gelagert. Die Beweglichkeit des Oberteils 10 gegenüber dem Unterteil 20 wird zusätzlich durch die Sperreinrichtung 30 beeinflusst, die zwischen der Lageraufnahme 12 des Oberteils und dem Sperrbereich 36 am Unterteil 20 angeordnet ist. Die Sperreinrichtung 30 lässt, je nach Zustand des nicht dargestellten Betätigungselementes 40 eine Bewegung entweder in nur eine oder beide Richtungen zu, wobei die Formgebung des Sperrelementes beliebig ist. Anstelle eines Wälzlagers sind für das zentrale Lager 120 auch andere Lagertechnologien, insbesondere Gleitlager, denkbar.

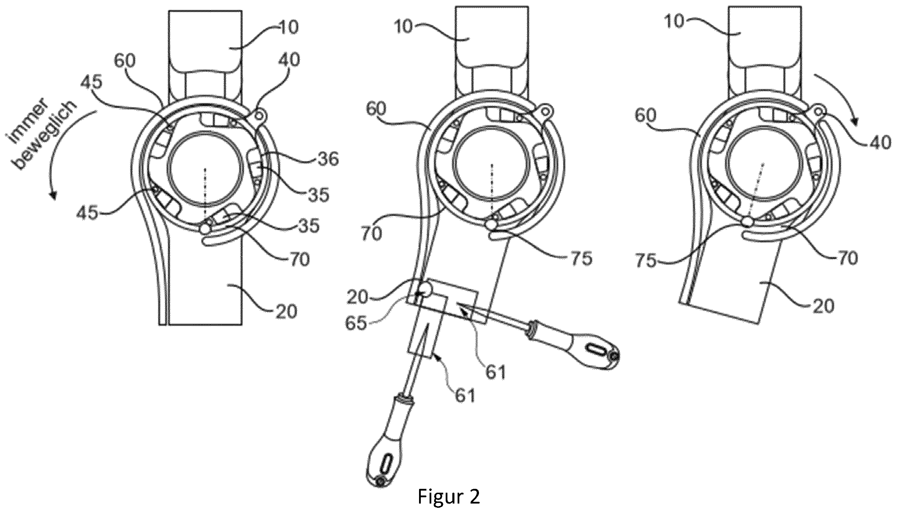

Eine Ausgestaltung ist in der Figur 2 gezeigt, bei der das Kopplungselement 70 als Ringelement ausgebildet ist. Eine Feder 60, die in dem dargestellten Ausführungsbeispiel als Blattfeder ausgebildet ist, ist an dem Ringelement 70 an einem Befestigungspunkt 75 festgelegt. In der linken Darstellung der Figur 2 ist das Oberteil 10 mit dem Unterteil 20 über die Sperreinrichtung 30 mit den Sperrelementen 35 verriegelt, eine freie Beweglichkeit im Uhrzeigersinn des Ringelementes 70 gegenüber dem Oberteil 10 ist nicht gegeben. Das Ringelement 70 stützt sich über die Feder 60 an dem Unterteil 20 ab. Aufgrund der elastischen Eigenschaften der Feder 60 ist auch bei einer aktivierten Sperre eine geringe Verschwenkung von Unterteil 20 zu Oberteil 10, wie in der mittleren Darstellung der Figur 2 gezeigt, möglich. Sobald über das Betätigungselement 40 die Sperrelemente 35 außer Eingriff gebracht werden, ist die Sperrwirkung aufgehoben und das Unterteil 20 bewegt sich zusammen mit dem Ringelement 70 relativ zu dem Oberteil 10. Dies ist in der rechten Darstellung der Figur 2 an dem verschwenkten Befestigungspunkt 75 zu erkennen. Da der Befestigungspunkt 75 bei freigeschalteter Sperreinrichtung in beide Richtungen frei beweglich ist, ist die Feder 60 in dieser Situation wirkungslos, wodurch eine ungehinderte Bewegung des Unterteils 20 gegenüber dem Oberteil 10 gegeben ist.

In der mittleren Darstellung der Figur 2 sind zwei Einstellvorrichtungen 61 für die Federvorspannung und die Federsteifigkeit der Feder 60 dargestellt. Über diese Einstellvorrichtungen 61 ist es möglich, den Auflagerpunkt das Auflager 65 des Federelementes 60 an dem Unterteil 20 unterschiedlich zu positionieren. Wird der Auflagerpunkt 65 in dem dargestellten Ausführungsbeispiel weiter nach links verlagert, erhöht sich die Federvorspannung, wird der Auflagerpunkt 65 weiter in Richtung auf die Schwenkachse 15 verschoben, erhöht sich die Steifigkeit aufgrund der verkürzten effektiven Federlänge.. Alternativ kann der Auflagerpunkt auch durch Einlegeteile, insbesondere Gleitschuhe oder keilförmige Abstandshalter, verlagert werden.

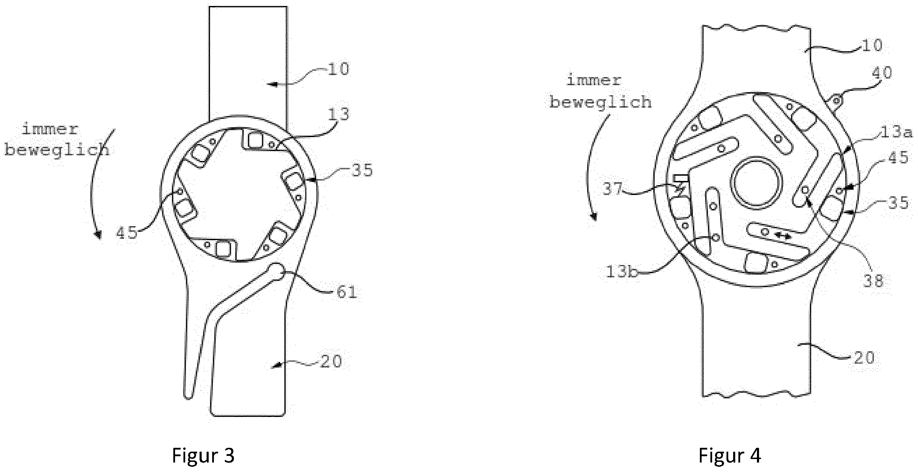

In der Figur 3 ist eine weitere Variante gezeigt, bei der die Verriegelung entsprechend dem Prinzip der Figuren 1 und 2 erfolgt. Die Sperre wirkt in diesem Beispiel bei einer Verdrehung des Unterteils 20 gegenüber dem Oberteil 10 im Uhrzeigersinn.

In dem Ausführungsbeispiel sind statt der oben gezeigten fünf Sperrelemente 35 sechs Sperrelemente 35 gleichmäßig um den Umfang der zylindrischen Innenfläche des Unterteils 20 angeordnet. Dementsprechend sind auch sechs Entriegelungsstifte 45 vorgesehen, die über eine nicht dargestellte Betätigungseinrichtung verdreht werden können, um die Sperrelemente 35 außer Eingriff zu bringen. Die Anzahl der Sperrelemente 35 und Entriegelungsstifte 45 kann davon abweichen und beliebig variieren. Um wie in der Figur 2 eine durch Federkraft limitierte Verschwenkung von Unterteil 20 zu Oberteil 10 auch bei einem verriegelten Gelenk und einer Sperrung der Verschwenkung um die Schwenkachse 15 zu ermöglichen, ist das Unterteil 20 in dem dargestellten Ausführungsbeispiel elastisch ausgebildet. Dies erfolgt durch die Einbringung eines Schlitzes 61 innerhalb des Unterteils 20 und die elastische Ausgestaltung entweder des Materials oder aber durch Anordnung eines elastischen Elementes in dem Schlitz 61. Eine Flexibilität von Oberteil 10 zu Unterteil 20 ist somit durch eine entsprechende flexible Ausgestaltung des Oberteils 10 oder Unterteils 20 gegeben. Der Schlitz 61 bildet in dem Unterteil 20 ein Gelenk oder Scharnier aus, wobei über die Breite des Schlitzes die maximale Verschwenkbarkeit von Oberteil 10 oder Unterteil 20 innerhalb des Gelenkes oder Scharnier durch den Schlitz 61 definiert wird. Die elastischen Eigenschaften des Unterteils 20 können durch unterschiedliche Materialien bzw. Geometrien elastischer Körper, die in den Schlitz 61 eingebracht werden können, variiert werden. Alternativ oder ergänzend kann auch das Oberteil 10, wie oben bereits erwähnt, mit einem Schlitz versehen oder elastisch ausgeführt werden. Durch eine elastische Ausführung sowohl des Oberteils 10 als auch des Unterteils 20 kann eine höhere elastische Auslenkung erreicht werden. Weiterhin werden in diesem Fall die Verformungen auf beide Komponenten aufgeteilt, was eine geringere Belastung der Materialien zur Folge hat.

Sind die Entriegelungsstifte 45 in Eingriff mit den Sperrelementen 35, ist eine freie Verschwenkbarkeit von Oberteil 10 zu Unterteil 20 möglich, ungeachtet einer flexiblen und elastischen Ausgestaltung des Unterteils 20 und/oder des Oberteils 10. Mit einer solchen Ausgestaltung ist eine serielle Anordnung einer Elastizität und Flexibilität zusammen mit einer Verriegelung in einer Schwenkrichtung bei einer permanenten Beweglichkeit in der entgegengesetzten Schwenkrichtung möglich.

Eine weitere Variante ist in der Figur 4 gezeigt, bei der die Anlaufschrägen 13 in Gestalt von federnden Armen 13a ausgebildet sind. In dem dargestellten Ausführungsbeispiel sind fünf Arme 13a ausgebildet, die sich bis an die Innenwand des Oberteils 10 erstrecken, aber auch kürzer sein können. In dem dargestellten Ausführungsbeispiel ist in dem Oberteil 10 eine zylindrische Reibfläche und damit der Sperrbereich 36 für die Sperrelemente 35 vorgesehen. Zwischen den Anlaufschrägen 13 der Arme 13a sind die Sperrelemente 35 angeordnet, denen Entriegelungsstifte 45 zugeordnet sind, die wieder verdrehbar um die Schwenkachse 15 an dem Betätigungselement 40 gelagert sind, um eine freie Verschwenkbarkeit in beiden Schwenkrichtungen zu ermöglichen. Sind die Entriegelungsstifte 45 nicht in Kontakt mit den Sperrelementen 35, so ist eine freie Bewegung des Unterteils 20 gegenüber dem Oberteil 10 nur entgegen dem Uhrzeigersinn möglich. In entgegengesetzter Richtung bewirken die Sperrelemente 35 eine Verbiegung der federnden Arme 13a, wodurch ein elastischer Widerstand realisiert wird. Die elastische Verformung der federnden Arme 13a kann durch einen definierten Auflagerpunkt 13b begrenzt werden, wodurch die elastische Kraft und damit das elastisch aufbringbare Moment um die Drehachse limitiert und bei höheren Momenten eine weitere Bewegung gesperrt wird. Der Auflagerpunkt 13b kann als Begrenzung durch entsprechende Formgebung in das Bauteil, das auch die Federelemente beinhaltet, integriert werden.

Den federnden Armen 13a sind in der dargestellten Ausführungsform an der Innenseite Stifte als Auflager 38 zugeordnet, die entlang der Arme 37 verschwenkbar oder verstellbar gelagert sind, was durch den Doppelpfeil angedeutet ist. Die Stifte als Auflager 38 können auch nicht vorgesehen sein. Werden die Stifte 38 weiter in Richtung auf das Ende des Schlitzes unterhalb der Arme 13a verlagert, verringert sich die Steifigkeit der Arme 13a. Werden die Stifte 38 weiter an das äußere Ende der Arme 13a verlagert, erhöht sich die Steifigkeit der Arme 13a. Die Arme 13a arbeiten somit als Biegebalken, deren Elastizität einstellbar ist. Zusätzlich zu der Elastizität der Arme 13a können die elastischen Elemente 37 zur Beaufschlagung der Sperrelemente 35 in Richtung auf eine Schließstellung vorgesehen sein. Durch die elastischen Elemente 37, die in Umfangsrichtung oder entlang der Längserstreckung der Arme 37 wirken, wird ein gleichmäßiger Anpressdruck der Sperrelemente 35 an den Sperrbereich 36 oder die Innenwand des Oberteils 10 sowie ein Toleranz- und Verschleißausgleich erreicht. Die elastische Ausgestaltung der Arme 13a unterstützt diesen Effekt, darüber hinaus wird das Abbremsen bei einem blockierten Gelenk abgefedert, wenn sich die Arme 13a geringfügig aufgrund ihrer Elastizität verformen.

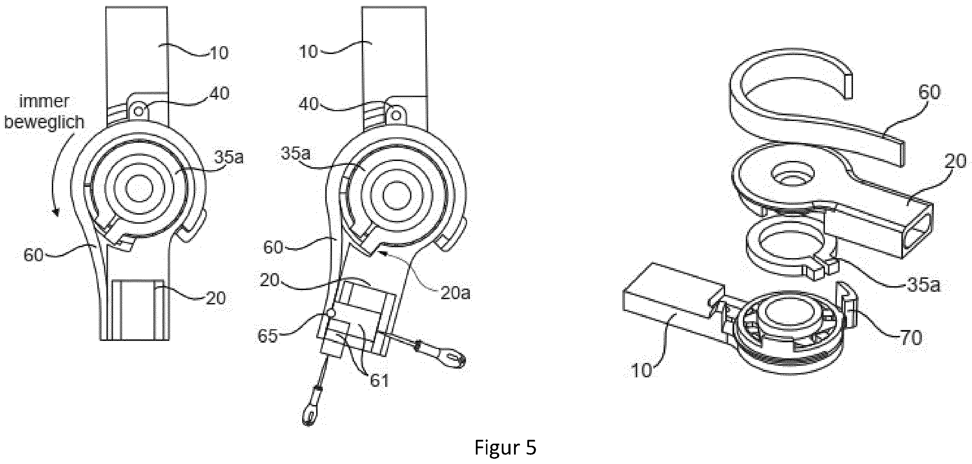

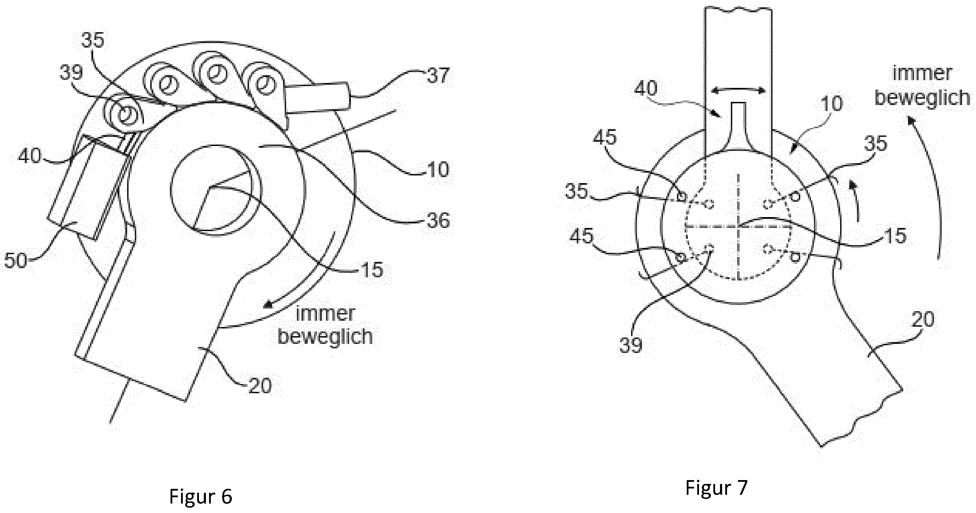

Figur 5 zeigt eine Variante mit einem Klemmring 35a der auch als Überlastschutz fungiert. Wieder ist eine Bewegung des Kopplungselementes 70 zur Federbefestigung gegenüber dem Oberteil 10 nur entgegen dem Uhrzeigersinn möglich. Im Uhrzeigersinn ist sie gesperrt, sofern das Kopplungs- oder Ringelement 70 nicht über den Betätigungsmechanismus 40 zur Bewegung freigegeben ist. Bei normaler Belastung ist der Klemmring 35a nur in losem Kontakt mit dem Oberteil 10, sodass eine Relativbewegung möglich ist. Bei hoher Belastung wird der Klemmring 35a durch den Kontakt des Unterteils 20 mit dem Klemmring 35a an der Stelle 20a festgezogen, wie in der mittleren Abbildung gezeigt, wodurch eine zusätzliche Kraftübertragungsstelle zwischen dem Oberteil 10 und dem Unterteil 20 entsteht. Über den belastungsabhängigen zusätzlichen Kraftschluss können die Sperrelemente 35 auf geringere Lasten, und damit kompakter, ausgelegt werden, weil gelegentlich auftretende Überlasten abgefangen werden. Darüber hinaus sind in Figur 6 Vorrichtungen 61 zur Einstellung der Federeigenschaften dargestellt. Dank dieser Vorrichtungen kann der Auflagerpunkt 65 der Feder 60 am Unterteil 20 verschoben werden, wodurch die Vorspannung und Steifigkeit der Feder 60 angepasst werden können. Über die Verstelleinrichtungen 61 können die Federvorspannung und die Steifigkeit wie oben beschrieben eingestellt werden. Dies kann beispielsweise über eine Schraube, einen Exzenter, Einlegeteile oder eine andere Vorrichtung oder Methode erfolgen. Auch eine motorische Verstellung ist denkbar.

In der Figur 6 ist eine weitere Variante gezeigt, bei der das Oberteil 10 nur schematisch dargestellt ist. Auf einer kreisförmigen Außenfläche des Unterteils 20 sind exzentrisch zu der Schwenkachse 15 gelagerte Bremskeile als Sperrelemente 35 an mit dem Oberteil 10 Achsen 39 gelagert. Die einzelnen Bremskeile sind geschuppt übereinander angelegt. Um Toleranzen und Verschleiß auszugleichen, können die einzelnen Bremskeile auch über elastische Elemente verbunden werden. An dem ganz rechten Bremskeil ist ein Federelement oder elastisches Element 37 angeordnet, das die Bremskeile in Richtung auf den äußeren Umfang des kreisförmigen Endstückes des Unterteils 20 drückt. Der äußere Umfang des Endstückes des Unterteils 20 bildet den Sperrbereich 36 oder die Sperrfläche aus, die mit den Bremskeilen in Eingriff tritt. Die Auflageflächen der Bremskeile liegen neben einer Verbindungslinie der jeweiligen Achse 39 zu der Schwenkachse 15, sodass eine Schwenkbewegung des Unterteils 20 nur in einer Schwenkrichtung möglich ist, in dem dargestellten Ausführungsbeispiel nur in Uhrzeigersinn. Wird das Unterteil 20 entgegen dem Uhrzeigersinn um die Schwenkachse 15 verlagert, verkeilen sich die Sperrelemente 35 an der Auflagefläche bzw. dem Sperrbereich 36 und verhindern eine weitere Drehung. Um diese Sperrwirkung aufzuheben, ist ein Betätigungselement 40 in Gestalt eines Schiebers vorgesehen, der unterhalb der Achse 39 des linken Sperrelementes 35 ansetzt und dieses von der Sperrfläche oder dem Sperrbereich 36 abhebt. Aufgrund der geschuppten oder elastisch verbundenen Anordnung der vier Sperrelemente 35 werden alle Sperrelemente 35 angehoben und eine Schwenkbewegung freigegeben. Das Betätigungselement 40 wird über einen Aktuator 50, beispielsweise ein Hubmagnet, Magnetaktuator, ein Piezostapel, ein Motor oder einen anderen Antrieb betätigt. Die Betätigung kann über einen Sensor oder mehrere Sensoren gesteuert erfolgen oder über einen Schalter manuell und individuell aktiviert werden. Alternativ zu der geschuppten Ausführung der Bremskeile, durch die sowohl die Kraft des Aktuators 50 als auch die Kraft des Vorspannelements 37 zwischen den Bremskeilen aufgeteilt wird, können diese auch getrennt voneinander ausgeführt sein, wobei dann für jeden Keil individuell eine Struktur zur Übertragung der Kraft des Aktuators 50 bzw. ein Vorspannelement 37 vorgesehen wird.

In der Figur 7 ist eine weitere Variante dargestellt, bei der statt keilförmiger Sperrelemente 35 Bügel schwenkbar an dem Oberteil 10 gelagert sind. Die Bügel 35 greifen außen um das Unterteil 20 herum, wobei die Lagerung 39 exzentrisch zu der Schwenkachse 15 ist. Das Unterteil 20 ist bei einer Verschwenkung entgegen dem Uhrzeigersinn frei beweglich. Bei einer Verschwenkung im Uhrzeigersinn sperren die Bügel als Sperrelemente 35 eine weitere Bewegung, da sie aufgrund der exzentrischen Lagerung und der Führung an der äußeren Oberfläche des Unterteils 20 mit Reibung bei einer Verschwenkung im Uhrzeigersinn gegen die Außenseite des Unterteils 20 gepresst werden und eine Verriegelung bewirken. Das Betätigungselement 40 mit den Entriegelungsstiften 45 arbeitet wie oben beschrieben, bei einer Verdrehung des Betätigungselementes 40 entgegen dem Uhrzeigersinn werden die Bügel 35 vom Unterteil 20 abgehoben. Dadurch erfolgt eine Entsperrung, sodass eine freie Beweglichkeit von Oberteil 10 zu Unterteil 20 in beiden Richtungen gewährleistet ist. Durch eine elastische Ausführung der Bügel 35 kann bei einem gesperrten Gelenk ein elastisches Verhalten entgegen der Sperrrichtung erzielt werden.

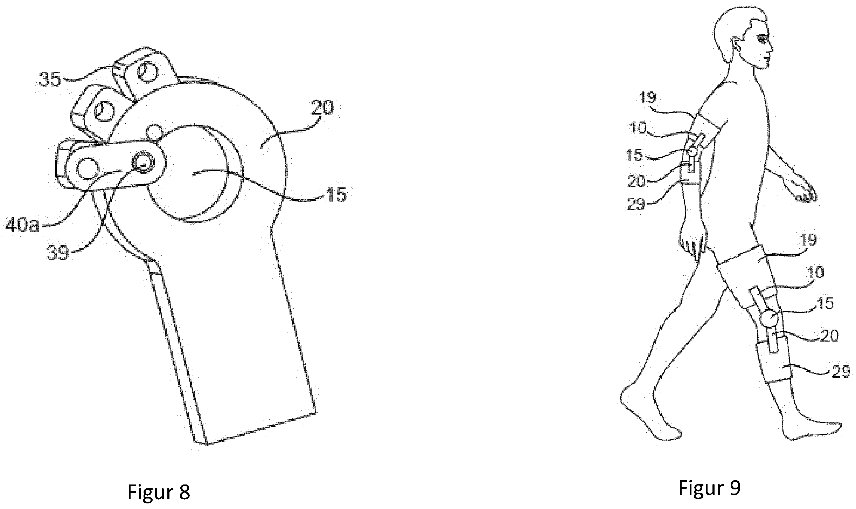

In der Figur 8 ist eine weitere Variante dargestellt, bei der der Bügel durch mit dem Betätigungselement 40 verbundene Kraftübertragungsstrukturen 40a, die mit den Sperrelementen 35 , z.B. Bremsbeläge, verbunden sind, ersetzt sind. Diese sind über Lagerstellen 39 exzentrisch gegenüber der Drehachse 15 an dem nicht dargestellten Oberteil 10 gelagert. Verschwenkt das Unterteil 20 in dem Uhrzeigersinn, so heben aufgrund der Reibkräfte über die Kraftübertragungsstruktur 40a die Sperrelemente 35 vom Unterteil 20 ab und eine freie Verschwenkbarkeit ist gegeben. In umgekehrter Schwenkrichtung werden die Sperrelemente 35 auf den Außenumfang des Unterteils 20 gepresst und eine Sperre bewirkt. Über ein mit der Kraftübertragungsstruktur 40a verbundenes, nicht dargestelltes Betätigungselement können die Sperrelemente 35 permanent von dem Unterteil 20 abgehoben werden, wodurch eine freie Bewegung in beide Schwenkrichtungen ermöglicht wird. Vorteilhafterweise werden auch hier die Sperrelemente 35 oder Kraftübertragungsstrukturen 40a mit einer geringen Vorspannkraft in Richtung auf das Unterteil 20 beaufschlagt, um ein sicheres Sperren und einen Verschleißausgleich zu gewährleisten. Durch eine elastische Ausführung der Kraftübertragungsstrukturen 40a kann ein elastisches Verhalten in Sperrrichtung erzielt werden.

In der Figur 9 sind zwei Anwendungsbeispiele der orthopädietechnischen Gelenkeinrichtung in Gestalt von Knieorthesen und Ellenbogenorthesen gezeigt. Beide Orthesen weisen ein Oberteil 10 und ein Unterteil 20 auf, die gelenkig um die Schwenkachse 15 aneinander gelagert sind. Das Oberteil 10 ist mit dem ersten Befestigungselement 19, beispielsweise einer Manschette, einer Schale oder Gurten an dem Oberarm festgelegt, während das Unterteil 20 über ein zweites Befestigungselement 29 an dem Unterarm festgelegt ist. Das zweite Befestigungselement 29 ist geeignet ausgebildet, beispielsweise ebenfalls als Schale, Teilschale, Manschette oder dergleichen. Zwischen dem Oberteil 10 und dem Unterteil 20 ist die Gelenkeinrichtung mit einer Sperreinrichtung, wie sie anhand der vorherigen Figuren beschrieben worden ist, angeordnet.

Mit allen orthopädietechnischen Gelenkeinrichtungen, wie sie oben dargestellt worden sind, ist eine mechanische Sicherung gegen ein Einbeugen in einer Richtung gegeben, sofern keine Maßnahmen getroffen werden, die die Sperrwirkung in der vorgegebenen, kritischen Schwenkrichtung aufhebt. Die Sperrung bleibt auch unter Last bestehen und wird bei einer sich vergrößernden Last durch eine positive Rückkopplung verstärkt. Damit bleibt die Sperrung auch erhalten, wenn bei elektronischen Komponenten einen Ausfall aufgrund eines Fehlers oder mangelnder Energieversorgung eintritt.

Alternativ können die Sperrelemente mit einer Vorspannkraft in die andere Richtung beaufschlagt werden, sodass sie aktiv in Eingriff gebracht werden müssen. In dieser Konfiguration ist das Gelenk im energielosen, nicht aktuierten Zustand frei. Für eine Anwendung in prothetischen oder orthetischen Kniegelenken kann dies unerwünscht sein. Bei einer Anwendung in Ellbogengelenken ist ein solches Verhalten aber vorteilhaft, da damit ein ungehindertes Schwingen des Armes, z.B. beim Gehen, sichergestellt werden kann und die Sperre nur aktiviert wird, wenn sie tatsächlich benötigt wird.

Ein Aktuator oder eine Betätigungseinrichtung dient zur Aufhebung der Friktion und damit auch eine Aufhebung der Sperrung, wenn die Sperrung nicht mehr benötigt wird. Vorteilhafterweise führt eine Sperrung zu einer Aktivierung einer seriell angeordneten Federeinrichtung, womit eine stoßartige Belastung der Sperre abgefedert werden kann. Darüber hinaus kann so eine elastische Zwischenspeicherung von Bewegungsenergie des Anwenders, z.B. während einer Standphasenflexion beim Gehen, realisiert werden. Die Sperrung der Gelenkeinrichtung in einer Bewegungsrichtung oder Schwenkrichtung ist in jeder Stellung möglich, eine freie Verschwenkbarkeit in entgegengesetzte Richtung ist immer gegeben.

Eine Eigenschaft all dieser Ausführungen ist, dass sie, sofern keine Belastung entgegen der Sperrrichtung anliegt, mit geringer Aktuatorkraft in den freien Zustand umgeschaltet werden können. Bei Belastung in Sperrrichtung steigt die hierfür erforderliche Schaltkraft stark an. Dies kann als Sicherheit gegen unbeabsichtigtes Umschalten unter Last genutzt werden, indem beispielsweise die Kraft des Aktuators zur Betätigung der Umschaltung begrenzt wird. Dadurch kann die Steuerung einer auf der Gelenkeinrichtung basierenden Orthesen- oder Prothesengelenks vereinfacht werden. Aufgrund dieser Eigenschaft kann in vielen Fällen auf eine zusätzliche Sensorik zur Messung des Belastungszustands verzichtet werden, da dieser auch z.B. über die Energieaufnahme oder den Zustand des Aktuators abgeschätzt werden kann.

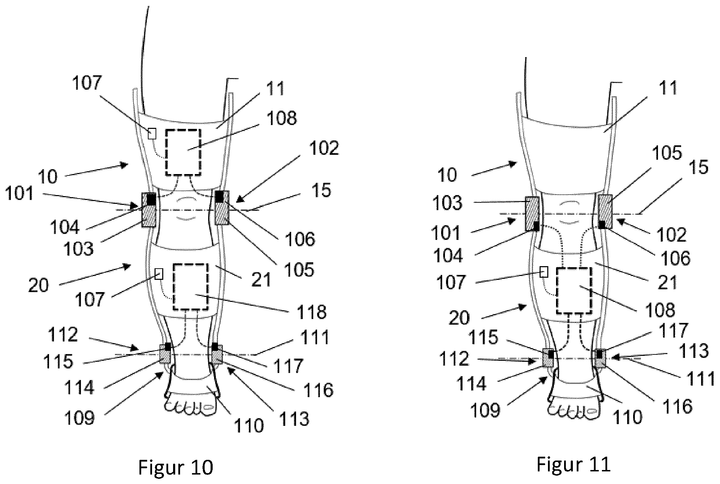

Figur 10 zeigt in einer Frontalansicht eine orthopädietechnische Gelenkeinrichtung als Teil einer sogenannten KAFO (knee-ankle-foot orthosis) im angelegten Zustand an einem rechten Bein. Das Oberteil 10 weist ein schalenartiges Befestigungselement in Form einer Oberschenkelschale 11 auf, das an einem Oberschenkel festgelegt wird. Die Festlegung erfolgt mit wiederholt lösbaren Befestigungselementen wie Gurten, Schnallen oder dergleichen. Das Unterteil 20 ist korrespondierend ausgebildet und weist eine Unterschenkelschale 21 zur Aufnahme des Unterschenkels auf. Auch das Unterteil 20 ist mit Gurten, Schnallen oder dergleichen an dem Bein wiederholt lösbar festgelegt. Die Schwenkachse 15 ist in dem dargestellten Ausführungsbeispiel auf der Höhe der natürlichen Kniegelenkachse angeordnet, sodass das Oberteil 10 und das Unterteil 20 bei einer Beugung und Streckung des Beines der Bewegung folgen können. Zwischen dem Oberteil 10 und dem Unterteil 20 ist lateral ein erster Aktuator 103 und medial ein zweiter Aktuator 105 angeordnet. Der erste Aktuator 103 bildet ein erstes Gelenk 101 aus und der zweite Aktuator 105 ein zweites Gelenk 102. Alternativ dazu kann der erste Aktuator 103 an einem ersten Gelenk 101 und der zweite Aktuator 105 an einem zweiten Gelenk 102 angeordnet sein. Die Aktuatoren 103, 105 beeinflussen die Verschwenkbarkeit des Unterteils 20 relativ zu dem Oberteil 10. Die Aktuatoren können dazu als motorischer Antrieb oder Dämpfer, insbesondere als Hydraulikdämpfer Pneumatikdämpfer, magnetorheologischer Dämpfer oder Rotationshydraulik ausgebildet sein, um die Beugebewegung und/oder die Streckbewegung mit einem Bewegungswiderstand zu beaufschlagen. Alternativ dazu können die Aktuatoren auch als Freigabe- und Verriegelungseinrichtung ausgebildet sein, um die Beugebewegung und/oder Streckbewegung zu sperren, wenn dies gewünscht oder erforderlich ist. Der erste Aktuator 103 weist ein erstes Einstellelement 104 auf und der zweite Aktuator 105 ein zweites Einstellelement 106. Über die Einstellelemente ist der Bewegungswiderstand des zugehörigen Aktuators gegen eine Verschwenkung des Unterteils 20 relativ zu dem Oberteil 10 um die Schwenkachse 15 einstellbar. Weiterhin ist ein Sensor 107 an dem Oberteil 10 angeordnet, der mit einer Steuereinheit 108 verbunden ist. Der Sensor 107 kann beispielsweise Raumlage, Kräfte, Momente, Beschleunigungen, Geschwindigkeiten, Winkel und andere Einflussgrößen oder Zustandsgrößen der orthopädietechnischen Gelenkeinrichtung erfassen und übermittelt Sensordaten an die Steuereinheit 108. Der Sensor 107 kann auch innerhalb des Oberteils 10 oder an einer anderen Stelle der orthopädietechnischen Gelenkeinrichtung angeordnet sein. Es können auch mehrere Sensoren 107 mit einer Steuereinheit 108 verbunden sein. Die Steuereinheit 108 ist mit dem ersten Einstellelement 104 und dem zweiten Einstellelement 106 verbunden und steuert diese synchronisiert in Abhängigkeit von den Sensordaten der Sensoren.

Unterhalb des Unterteils 20 ist ein Fußteil 109 angeordnet, das eine Fußschale 110 aufweist. Alternativ zu einer Fußschale kann auch eine Schiene oder Stütze vorgesehen werden. Zwischen dem Unterteil 20 und dem Fußteil 109 ist lateral ein dritter Aktuator 114 und medial ein vierter Aktuator 116 angeordnet. Der dritte Aktuator bildet ein drittes Gelenk 112 und der vierte Aktuator 116 ein viertes Gelenk 113 aus. Die Aktuatoren 114, 116 beeinflussen die Verschwenkbarkeit des Fußteils 109 relativ zu dem Unterteil 20 um die weitere Schwenkachse 111. Der dritte Aktuator 114 weist ein drittes Einstellelement 115 und der vierte Aktuator 116 ein viertes Einstellelement 117 auf. Über die Einstellelemente 115, 117 ist der Bewegungswiderstand des zugehörigen Aktuators gegen eine Verschwenkung des Fußteils 109 relativ zu dem Unterteil 20 um die weitere Schwenkachse 111 einstellbar. Das Unterteil 20 weist eine weitere Steuereinheit 118 auf, die mit einem Sensor 107, dem dritten Einstellelement 115 und dem vierten Einstellelement 117 verbunden ist. Die weitere Steuereinheit 118 steuert das dritte Einstellelement 115 und das vierte Einstellelement 117 synchronisiert in Abhängigkeit von Sensordaten der Sensoren 107. Das Sensor-Setup 107 kann bei mehreren Steuereinheiten 118 unterschiedlich ausgebildet sein.

In der Figur 11 ist eine Variante der Ausgestaltung gemäß Figur 10 dargestellt, bei der nur eine einzige Steuereinheit 108 und keine weitere Steuereinheit 118 vorgesehen ist. Die Steuereinheit 108 ist in dieser Ausgestaltung an dem Unterteil 20 angeordnet. Grundsätzlich ist es auch möglich, die Steuereinheit 108 an dem Oberteil 10 oder an dem Fußteil 109 anzuordnen. Nicht nur das erste Einstellelement 104 und das zweite Einstellelement 106 werden durch die Steuereinheit 108 angesteuert, sondern auch das dritte Einstellelement 115 und das vierte Einstellelement 117. Dabei ist es möglich, dass alle Einstellelemente synchronisiert in Abhängigkeit von Sensordaten der Sensoren 107 angesteuert werden. Alternativ dazu kann für die Einstellelemente jeder Schwenkachse 15, 111 eine separate synchronisierte Steuerung vorgesehen sein. So ist es beispielsweise denkbar, dass das erste Einstellelement 104 und das zweite Einstellelement 106 synchronisiert gesteuert werden und dass das dritte Einstellelement 115 und das vierte Einstellelement 117 synchronisiert gesteuert werden.

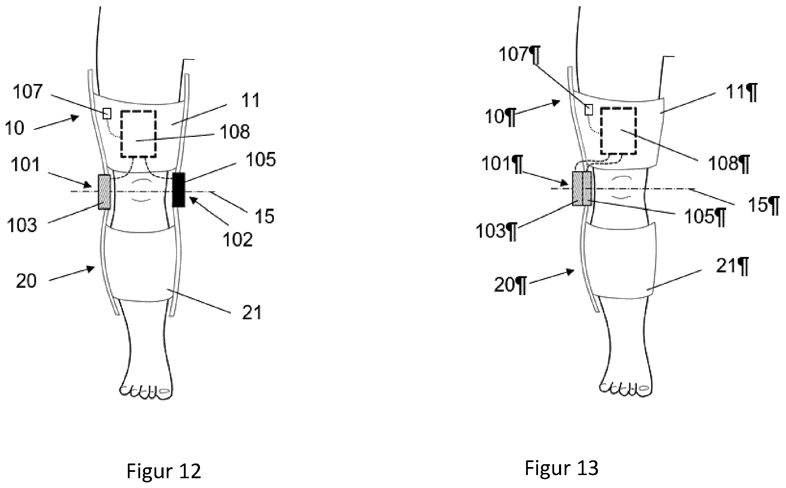

Eine Variante der orthopädietechnischen Gelenkeinrichtung in Form einer Knieorthese ist in der Figur 12 dargestellt. Die orthopädietechnische Gelenkeinrichtung besteht aus einem Oberteil 10 und einem Unterteil 20, das um die Schwenkachse 15 schwenkbar zu dem Oberteil 10 angeordnet ist. Das Oberteil 10 weist eine Oberschenkelschale 11 zur Aufnahme des Oberschenkels auf. Das Unterteil 20 weist korrespondierend dazu eine Unterschenkelschale 21 zur Aufnahme des Unterschenkels auf. Zwischen dem Oberteil 10 und dem Unterteil 20 ist lateral ein erster Aktuator 103 und medial ein zweiter Aktuator 105 angeordnet. Die beiden Aktuatoren 103, 105 wirken um die Schwenkachse 15. Der erste Aktuator 103 bildet ein erstes Gelenk 101 und der zweite Aktuator 105 ein zweites Gelenk 102 aus. Alternativ dazu ist der erste Aktuator 103 an einem ersten Gelenk 101 und der zweite Aktuator 105 an einem zweiten Gelenk 102 angeordnet. Der erste Aktuator 103 und der zweite Aktuator 105 basieren in diesem Ausführungsbeispiel auf einem unterschiedlichen mechanischen Wirkprinzip. Beispielsweise ist der lateral Aktuator 103 als motorischer Antrieb oder als hydraulische Widerstandseinrichtung, insbesondere als Rotationshydraulik ausgebildet, während der mediale Aktuator 105 als Freigabe- und/oder Verriegelungseinrichtung ausgebildet ist. Das Oberteil weist einen Sensor 107 und eine Steuereinheit 108 auf. Die nicht dargestellten Einstellelemente 104 und 106 werden durch die Steuereinheit 108 synchronisiert gesteuert.

Figur 13 zeigt eine Variante der in Figur 12 dargestellten Ausführung, bei der beide Aktuatoren 103, 105 lateral an dem Knie angeordnet sind. Der erste Aktuator 103 und der zweite Aktuator bilden daher ein gemeinsames Gelenk aus oder sind an diesem angeordnet. Durch die ausschließlich laterale Anordnung der Aktuatoren besteht auf der medialen Seite ein größerer Bewegungsfreiraum. Darüber hinaus können mediale Schienen entfallen, sodass eine leichtere Bauweise erzielt werden kann.

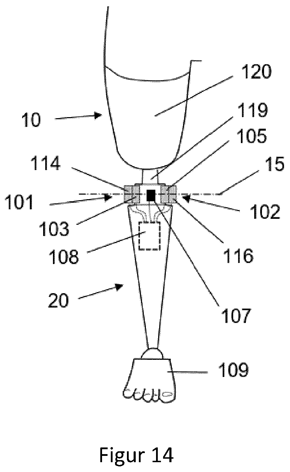

In der Figur 14 ist eine weitere Ausgestaltung dargestellt, bei der die orthopädietechnische Gelenkeinrichtung als Beinprothese ausgebildet ist. Das Oberteil 10 weist eine Aufnahme 120 für einen Beinstumpf und ein Anschlussmittel 119 beispielsweise in Form eines Pyramidenadapters auf. Das Unterteil 20 ist um die Schwenkachse 15 schwenkbar an dem Oberteil 10 angeordnet. An dem Unterteil 20 ist distal ein Fußteil 109 angeordnet, das als Fußprothese ausgebildet sein kann. Ein erster Aktuator 103 und ein dritter Aktuator 114 sind lateral zwischen dem Oberteil 10 und dem Unterteil 20 an der Schwenkachse 15 angeordnet. Auf der medialen Seite sind ein zweiter Aktuator 105 und ein vierter Aktuator 116 angeordnet. Die lateralen Aktuatoren 103, 114 bilden ein erstes Gelenks 101 aus und die medialen Aktuatoren 105, 116 bilden ein zweites Gelenk 102 aus. Weiterhin ist ein Sensor 107 an der Schwenkachse angeordnet. Alternativ kann der Sensor auch an anderer Stelle an dem Oberteil 10 oder dem Unterteil 20 vorgesehen sein. Der Sensor 107 kann beispielsweise Raumlage, Kräfte, Momente, Beschleunigungen, Geschwindigkeiten, Winkel und andere Einflussgrößen oder Zustandsgrößen der orthopädietechnischen Gelenkeinrichtung erfassen und übermittelt Sensordaten an die Steuereinheit 108. Zum Erfassen dieser Daten kann die orthopädietechnische Gelenkeinrichtung auch weitere Sensoren aufweisen. Die Aktuatoren 103, 105, 114, 116 weisen jeweils ein Einstellelement auf, das nicht gezeigt ist. Die nicht dargestellten Einstellelemente 104, 106, 115, 117 sind mit der Steuereinheit 108 verbunden und werden von dieser synchronisiert in Abhängigkeit von Sensordaten gesteuert. Durch die Anordnung mehrerer Aktuatoren auf der medialen und lateralen Seite kann die orthopädietechnische Gelenkeinrichtung im Rahmen eines modularen Systems an das Körpergewicht eines Benutzers angepasst werden. Bei einem geringen Körpergewicht des Benutzers kann es beispielsweise ausreichend sein, wenn nur der erste Aktuator 103 und der zweite Aktuator 105 vorgesehen werden. Bei einem höheren Körpergewicht des Benutzers kann ein dritter Aktuator 114 an dem ersten Aktuator 103 befestigt werden und ein vierter Aktuator 116 an dem zweiten Aktuator 105. Zur Lagesicherung und Befestigung der Aktuatoren können diese korrespondierende Formschlusselemente, wie z.B. eine Schwalbenschwanzverbindung oder eine Klauenkupplung, aufweisen. Alternativ oder ergänzend dazu kann eine kraftschlüssige Verbindung der Aktuatoren, z.B. durch Verschraubung oder Vernieten, und/oder eine stoffschlüssige Verbindung, z.B. durch Löten, Schweißen oder Kleben, vorgesehen werden. In einer weiteren Variante sind weitere Aktuatoren sowohl an der medialen als auch an der lateralen Seite angeordnet. Die elektrische Verbindung wird durch lösbare Kontakte oder Stecker sichergestellt. Grundsätzlich kann die Anzahl der Aktuatorelemente abhängig von den Patientenbedürfnissen beliebig variieren.

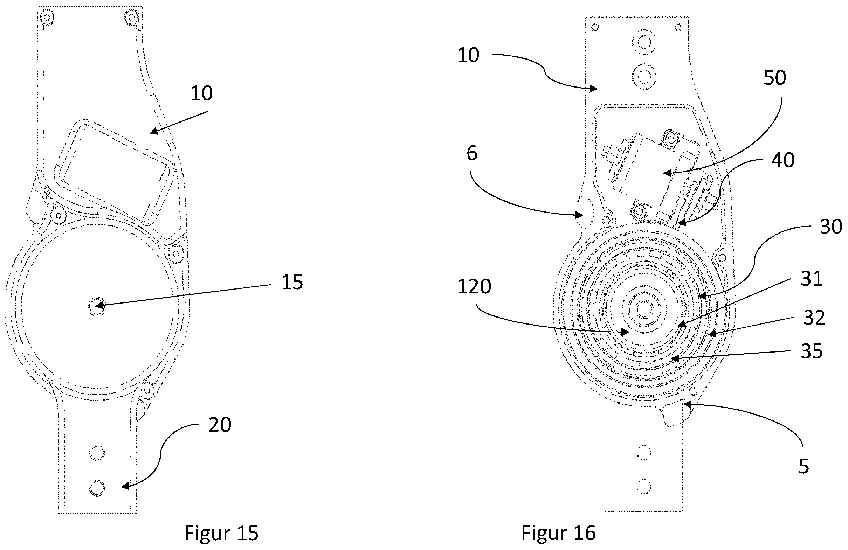

In den Figuren 15 und 16 ist eine weitere Variante der orthopädietechnischen Gelenkeinrichtung in Gestalt eines künstlichen Kniegelenkes dargestellt, beispielsweise eines künstlichen Orthesenkniegelenkes mit einem Oberteil 10 und einem Unterteil 20, die schwenkbar um eine Schwenkachse 15 aneinander gelagert sind. In der Figur 16, in der eine Abdeckung entfernt ist, ist neben einer zentralen Lagerung 120 die Sperreinrichtung 30 zu erkennen, die über ein Betätigungselement 40 in Gestalt eines Hebels und einen Aktuator 50 betätigt werden kann. An dem Oberteil 10 sind zudem ein Extensionsanschlag 5 und ein Flexionsanschlag 6 ausgebildet, die die maximalen Positionen des Oberteils 10 relativ zu dem Unterteil 20 definieren. Die Anschläge 5, 6 sind in dem dargestellten Ausführungsbeispiel fixiert, sie können grundsätzlich auch einstellbar oder verstellbar ausgebildet sein. In der dargestellten Position befindet sich die orthopädietechnische Gelenkeinrichtung in der maximal extendierten Stellung. Das Zentrallager 120 verbindet das Oberteil 10 mit dem Unterteil 20 und überträgt Lasten in Axialrichtung ebenso wie in Medialrichtung und Lateralrichtung. Ein innerhalb der Gelenkeinrichtung angeordneter Freilauf wird durch die zentrale Lagerung von diesen Belastungen entkoppelt und wirkt nur um die Gelenkachse 15 bzw. die Knieachse. Bei einer Bewegung des Innenringes 31 gegenüber dem Außenring 32 entgegen der Sperrrichtung, verkannten sich die jeweiligen Sperrelemente 35, sodass sie sich zwischen dem Innenring 31 und dem Außenring 32 verspreizen und einklemmen und eine Verriegelung in der gewünschten Sperrrichtung erfolgt. In der entgegengesetzten Richtung erfolgt keine Sperrwirkung durch die Sperreinrichtung 30, sodass in diese Richtung immer eine Verschwenkung möglich ist. Beispielsweise ist eine Extension stets möglich, eine Flexion wird immer gesperrt, es sei denn, dass der Aktuator 50 und das Betätigungselement 40 eine entsprechende Stellung eingenommen haben, um die Sperrelemente 35 außer Eingriff mit den entsprechenden Komponenten zu bringen. Über die Sperreinrichtung 30 und den Aktuator 50 wird über das Betätigungselement 40 das jeweilige Sperrelement 35 bzw. die Sperrelemente 35 außer Eingriff mit dem Außenring 32 oder dem Innenring 31 gebracht, sodass keine Verriegelung erfolgt.

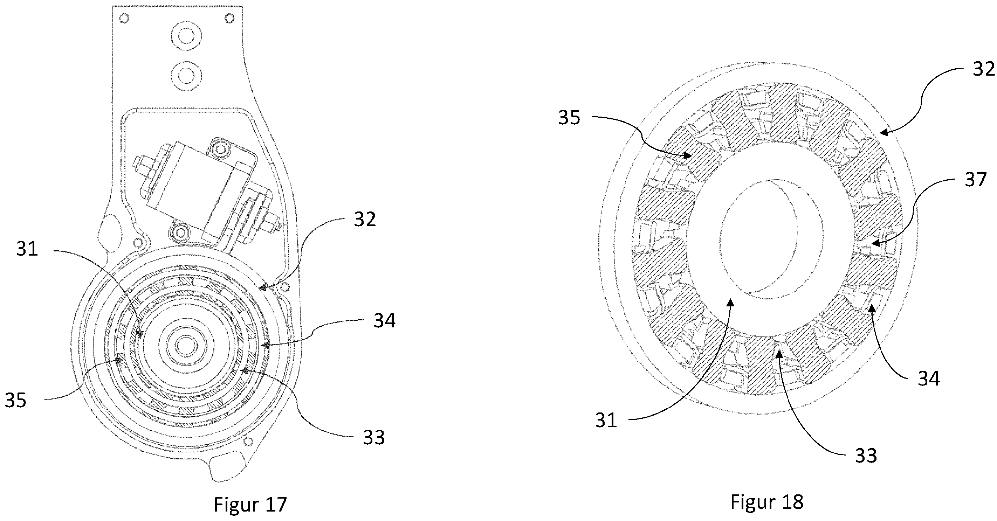

In der Figur 17 ist eine Detaildarstellung des Oberteils 10 mit dem schaltbaren Klemmkörperfreilauf dargestellt. Die Sperreinrichtung weist zwei Käfige 33, 34 auf, die zwischen dem Innenring 31 und dem Außenring 32 angeordnet sind. Der Innenkäfig 33 und der Außenkäfig 34 sind verdrehbar zueinander gelagert und jeweils mit dem Oberteil 10 und dem Betätigungselement 40 gekoppelt. Werden die beiden Käfige 33, 34 relativ zueinander verdreht, verdrehen sich die Sperrelemente 35, die zwischen dem Innenring 31 und dem Außenring 32 angeordnet und mit entsprechenden Eingriffskomponenten des Innenkäfigs 33 bzw. Außenkäfigs 34 in Eingriff gebracht sind. Die Eingriffskomponenten bewirken bei einer Relativverdrehung der Käfige zueinander, dass die Sperrelemente von dem Innenring 31 oder dem Außenring 32 abgehoben werden, wodurch eine Verschwenkbarkeit des Oberteils 10 relativ zu dem Unterteil 20 in beiden Richtungen gegeben ist. In der Figur 18 ist eine Schnittdarstellung durch die Sperreinrichtung mit dem schaltbaren Klemmkörperfreilauf gezeigt, aus der zu erkennen ist, dass durch Verdrehung des Innenkäfigs 33 in Uhrzeigerrichtung die Sperrelemente 35 von dem Innenring 31 abgehoben werden. Den Sperrelementen 35 ist ein Vorspannelement 37 in Gestalt einer Sperrelementefeder zugeordnet, die die Sperrelemente 35 in Richtung auf den Innenring 31 vorspannt, sodass bei Wegfall der Verdrehung automatisch eine Rückstellung oder eine Vorspannung der Sperrelemente 35 in Richtung auf den Innenring 31 erfolgt. Je nach Orientierung kann auch ein Abheben von dem Außenring 32 erfolgen.

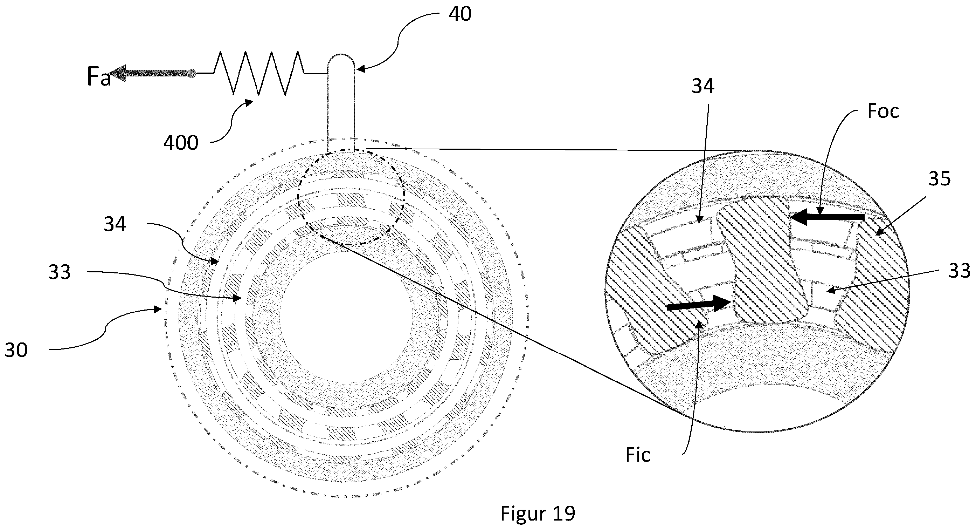

In der Figur 19 ist in einer Detaildarstellung die Umsetzung der Schaltung und Funktionsweise mit dem Doppelkäfig gemäß der Figuren 17 und 18 dargestellt. Das Betätigungselement 40 wird über einen nicht dargestellten Aktuator mit einer Betätigungskraft Fa beaufschlagt. Zwischen dem Aktuator und dem Betätigungselement 40 kann eine Schaltfeder 400 angeordnet sein. Das Betätigungselement 40 ist mit dem Außenkäfig 34 gekoppelt und bewirkt eine Verdrehung des Außenkäfigs 34 relativ zu dem Innenkäfig 33. In der rechten Detaildarstellung werden die aufgebrachten Kräfte dargestellt, die Kraft Foc durch den Außenkäfig wirkt in dem dargestellten Ausführungsbeispiel nach links, die entsprechende entgegenwirkende Abstützkraft Fic wirkt von dem Innenkäfig 33. Die Eingriffskomponenten sind jeweils zwischen den Sperrelemente 35 angeordnet. Die Schaltfeder 400 dient zum Ausgleich verformungsbedingter Bewegungen unter Last. Alternativ zu der Betätigungskraft Fa können die Käfige auch direkt mit einem Moment beaufschlagt werden.

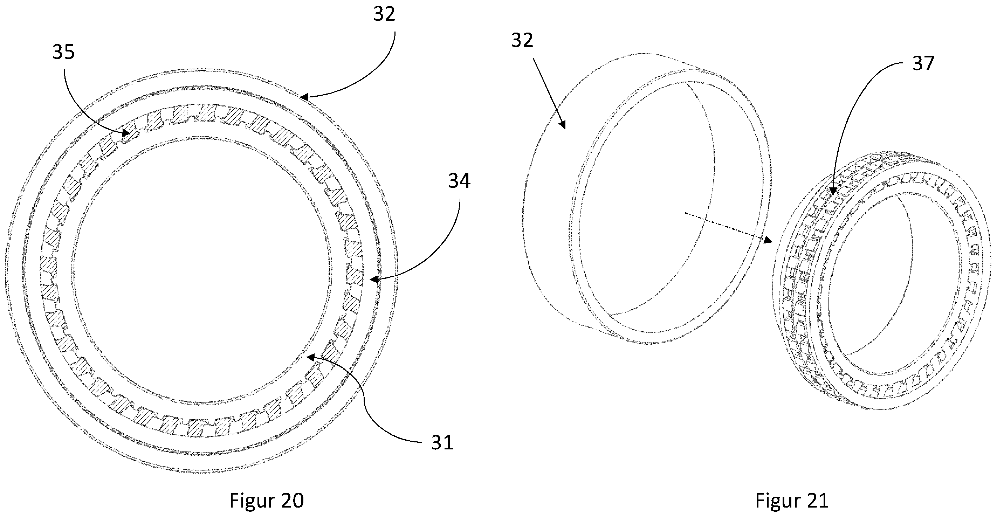

Eine Variante ist in den Figuren 20 und 21 gezeigt, wobei die Figur 20 eine Seitenansicht der Sperreinrichtung mit dem Freilauf darstellt. Die Sperrelemente 35 sind zwischen dem Innenring 31 und dem Außenring 32 an dem Innenring 31 gegen Verschiebung entlang des Umfanges gesichert und werden über einen Außenkäfig 34 außer Eingriff mit dem Außenring 32 gebracht, wenn eine freie Verschwenkbarkeit in beiden Richtungen gegeben sein soll. Dreht sich der Außenring 32 bei einem feststehenden Innenring 31 im Uhrzeigersinn, findet keine Sperrwirkung durch die Sperrelemente 35 statt, bei einer entgegengesetzten Bewegung ohne die Aktivierung des Außenringes 34 wird eine Verdrehung gesperrt. In der Figur 21 ist der Außenring 32 als geschlossener Ring mit einer wesentlichen glatten Innenfläche und einem glatten Innenumfang gezeigt, innerhalb dessen der Innenring 31 mit Sperrelementen 35, dem Außenkäfig 34 und dem Vorspannelement 37 angeordnet sind. Über das Vorspannelement 37 wird sichergestellt, dass die Sperrelemente 35 an dem Innenumfang des Außenringes 32 anliegen, sofern sich der Außenkäfig 34 nicht in der Freigabestellung befindet. Sinngemäß können die Funktionalitäten von Innenring 31 und Außenring 32 auch vertauscht werden, d. h., dass die Sperrelemente 35 an dem Außenring 32 gegenüber Verschiebung entlang des Umfanges gesichert werden, wobei der Innenring 31 dann glatt ausgeführt sein muss.

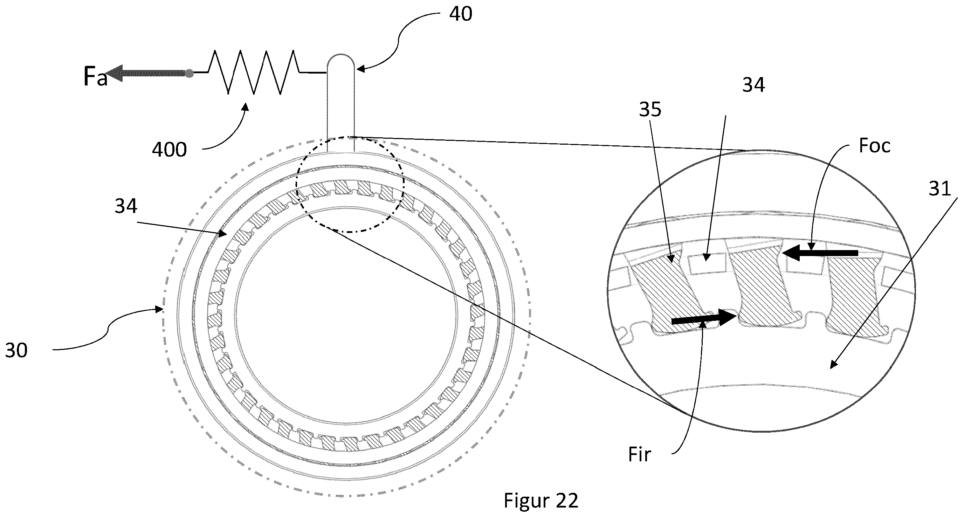

Die Betätigung der Ausführungsform gemäß Figuren 20 und 21 ist in der Figur 22 schematisch dargestellt. Auch hier wird eine Schaltkraft Fa unter Zwischenschaltung der optionalen Schaltfeder 400 von einem Aktuator aufgebracht und auf das Betätigungselement 40 übertragen, das mit dem Außenkäfig 34 gekoppelt ist. Die Sperrelemente 35 sind in dem Innenring 31 in Ausnehmungen gelagert und können verschwenkt werden. Wird der Außenkäfig 34 nach links in Uhrzeigerrichtung bewegt, wird eine Kraft Foc auf die Sperrelemente 35 durch die Eingriffskomponenten des Außenkäfigs 34 ausgeübt, sodass die Sperrelemente von dem Außenring abgehoben werden. Dadurch ist eine freie Bewegung in beiden Richtungen möglich.

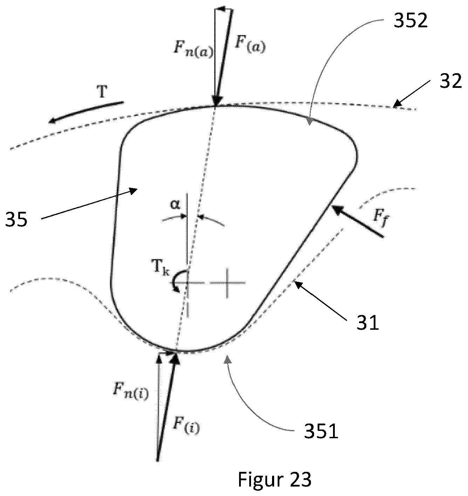

In der Figur 23 ist eine Ausführungsform der Lagerung der Sperrelemente 35 an dem Innenring 31 schematisch dargestellt. Das Sperrelement 35 liegt an einer entsprechenden Ausnehmung oder Lagerungsstelle des Innenringes 31 an und weist eine abgerundete oder kreisförmige Unterkontur 351 auf. Die Unterkontur 351 korrespondiert im Wesentlichen mit der Formgebung der Aufnahme in dem Innenring 31. Durch die abgerundete Form wird die Kontaktfläche zwischen dem Sperrelement 35 und dem Innenring 31 erhöht, wodurch die Flächenpressungen und damit die Kontaktspannungen reduziert werden. Die Oberkontur 352 weist einen größeren Radius als die Unterkontur 351 auf und liegt an dem Innenumfang des Außenringes 32 an. Je größer der Radius der Oberkontur 352 ist, desto größer ist die Kontaktfläche mit dem Außenring 32.

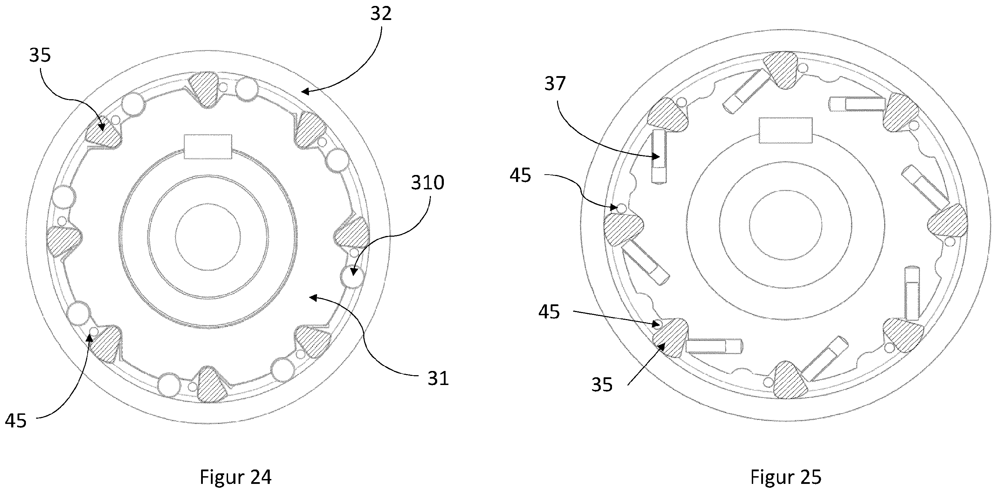

In der Figur 24 ist eine Schnittdarstellung der Sperreinrichtung mit dem Innenring 31 und dem Außenring 32 dargestellt. Der Innenring 31 weist Ausnehmungen auf, in denen die Sperrelemente 35 mit der abgerundeten unter Kontur 351 angeordnet sind. Jedem der Sperrelemente 35 ist ein Entriegelungsstift 45 zugeordnet, der über das nicht dargestellte Betätigungselement 40 verlagerbar ist. Bei der entsprechenden Betätigung des Entriegelungsstiftes 45 oder der Entriegelungsstifte 45 werden die Sperrelemente 35 von dem Außenring 32 abgehoben, sodass eine freie Beweglichkeit des Oberteils 10 relativ zu dem Unterteil 20 in beiden Schenkelrichtungen gegeben ist. Der Innenring 31 ist über Rollenlager 310 an dem Außenring 32 gelagert, um bei einer zugelassenen Drehbewegung eine ausreichende Abstützung der Komponenten aufeinander zu gewährleisten.

In der Figur 25 sind die Sperrelemente 35 wie in der Figur 24 gelagert und über Entriegelungsstifte 45 außer Eingriff mit dem Außenring 32 bringbar. Darüber hinaus sind Vorspannelemente 37 als Federn in Führungen in dem Innenring 31 angeordnet, die die Sperrelemente 35 gegen den Außenring 32 drücken und so einen Kontakt damit eine Verklemmung bei Verdrehung in Sperrrichtung sicherstellen. Durch die Entriegelungsstifte 45 können die Sperrelemente 35 gegen die Kraft der Vorspannelemente 37 verdreht werden, wodurch der Außenring 32 in beide Richtungen drehbar freigegeben wird.

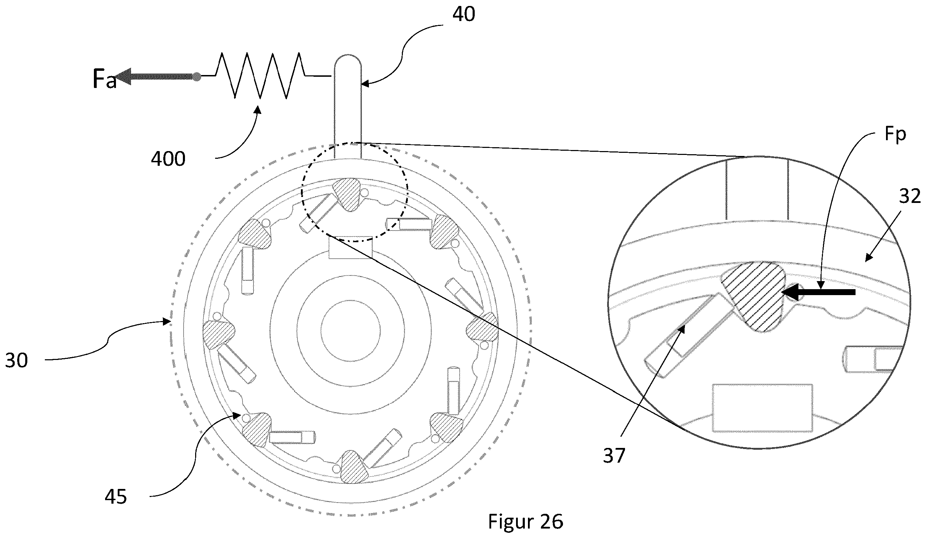

Der Betätigungsmechanismus der Vorrichtung gemäß Figur 25 ist in der Figur 26 dargestellt. Auch hier wird die Schaltkraft Fa über die optionale Schaltfeder 400 ausgeglichen oder begrenzt und eine elastische Nachgiebigkeit des Betätigungselementes 40 ermöglicht. Bis Betätigungselement 40 werden die Entriegelungsstifte 45 bewegt. Die dabei wirksame Kraft Fp ist in der vergrößerten Darstellung gezeigt. Sobald die Kraft Fp durch den Entriegelungsstift 45 größer als die Vorspannkraft des Vorspannelementes 37 ist, werden die Sperrelemente 35 von dem Außenring 32 abgehoben, sodass der Außenring 32 in beiden Richtungen frei schwenkbar ist.

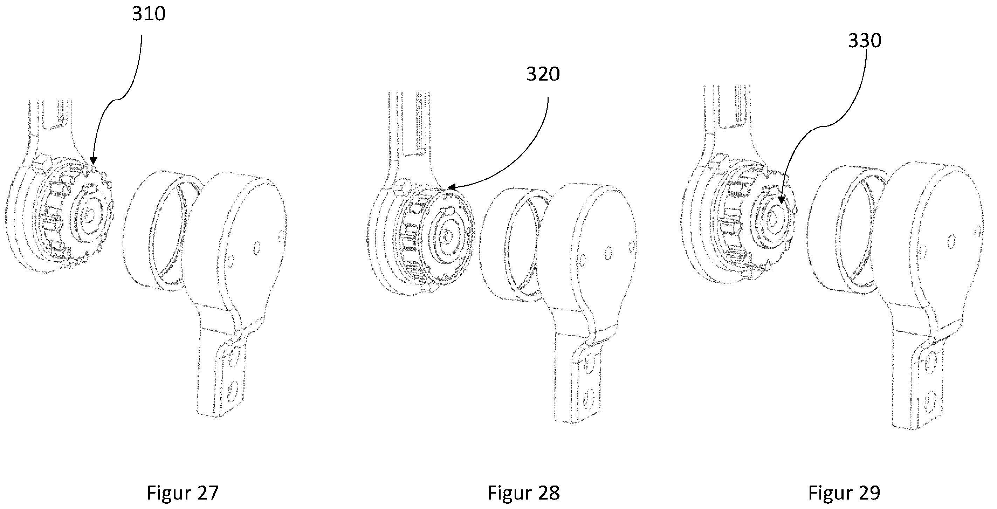

Die Figuren 27 bis 29 zeigen unterschiedliche Lagerungskonzepte, in der Figur 27 sind Innenring und Außenring über Rollenlager 310 aneinander gelagert, sodass ein integriertes Rollenlager in der Sperreinrichtung 30 vorhanden ist. In der Figur 28 wird ein integriertes Gleitlager 320 an dem Innenring 31 befestigt, sodass der Außenring 32 an dem Gleitlager des Innenringes 31 gelagert ist. In der Figur 29 ist eine Sperreinrichtung mit einer zentralen Lagerung über ein Gleitlager 330 auf der Hauptachse dargestellt.

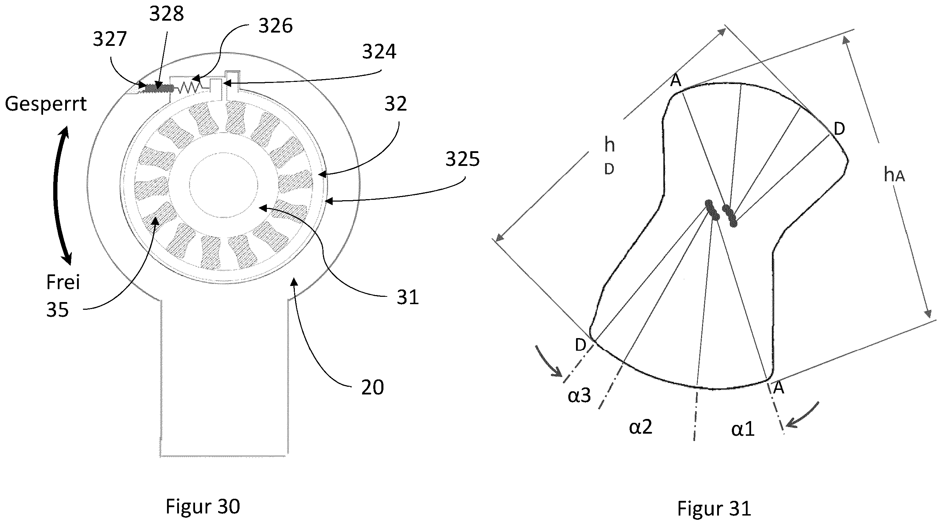

Eine Variante der Erfindung ist in der Figur 30 dargestellt, bei der der Außenring 32 geschlitzt und gegen eine Verdrehung gesichert in dem Unterteil 20 gelagert ist. Alternativ zu der Lagerung in dem Unterteil 20 kann der Außenring 32 auch in dem Oberteil 10 angeordnet oder ausgebildet sein. Der Außenring 32 verfügt über einen Schlitz 324, über den es möglich ist, den Innenumfang des Außenringes 32 einzustellen. Die Sicherung gegen das Verdrehen innerhalb des Unterteils 20 erfolgt beispielsweise über eine Ausnehmung, in die ein radialer Vorsprung des Außenringes 32 eingeführt ist. Alternative Befestigungseinrichtungen oder Befestigungsmöglichkeiten wie Schweißen, Löten, Kleben, Verklemmen o. ä. sind ebenfalls möglich. Zwischen dem Außenumfang des Außenringes 32 und dem Gehäuse oder der Lagerungsstelle in dem Unterteil 20 ist ein Ringspalt 325 vorhanden, sodass der Außenumfang und damit auch der Innenumfang des Außenringes 32 durch Vergrößerung oder Verkleinerung des Schlitzes 324 verändert werden können. Die Veränderung der Breite des Schlitzes 324 erfolgt über die Vorspanneinrichtung 327, bei der eine Vorspannfeder 326 beispielsweise über eine Vorspannschraube 328 vorgespannt wird. Über die Vorspannfeder 326 ist es möglich, eine gewisse Elastizität auch bei der Positionierung der Sperrelemente 35 in der Sperrstellung zu ermöglichen. Selbst in der gesperrten Stellung kann bei einer aufgebrachten Last eine geringfügige Verlagerung von Oberteil zu Unterteil stattfinden. Die Breite des Schlitzes 324 ist über die Vorspanneinrichtung 327 und gegebenenfalls die Feder 326 einstellbar. Dadurch kann auch bei einem geschlossenen Schlitz 324 über eine Kraft, die in Richtung der Vergrößerung des Schlitzes 324 wirkt, eine Elastizität der Sperreinrichtung 30 entgegen der Sperrrichtung verändert und eingestellt werden. Insbesondere kann durch den Einsatz einer Vorspannfeder 326 mit einer progressiven Federkennlinie die Steifigkeit entgegen der Sperreinrichtung eingestellt werden.

Eine weitere Möglichkeit zur Veränderung und Einstellung der Elastizität entgegen der Sperrrichtung kann durch eine entsprechende Geometrie der Sperrelemente 35 erfolgen. Ein solches Sperrelement ist in der Figur 31 dargestellt, das in dem dargestellten Ausführungsbeispiel drei Elastizitätsbereiche α1, α2, α3 aufweist, die unterschiedliche Krümmungsradien und unterschiedliche Krümmungsmittelpunkte hinsichtlich ihrer Außenkontur aufweisen. Die entsprechenden Elastizitätsbereiche sind auf den Oberkonturen und Unterkonturen aufgetragen, h_{A} ist dabei die anfängliche oder initiale Spaltweite zwischen den Punkten A auf den oberen und unteren Konturen, wobei h_{D} die finale Spaltweite zwischen den Punkten D auf den oberen und unteren Konturen darstellt. Die Konturgebungen der äußeren und inneren Kontaktfläche des Sperrelementes 35 sind flexibel wählbar. Insbesondere können die Elastizitätsbereiche über die Konturgebung der äußeren Kontaktfläche realisiert werden, wobei an der inneren Kontaktfläche eine weitgehend kreisförmige Kontur, wie in der Figur 23 dargestellt ist, realisiert werden kann.

Die Elastizität der Sperreinrichtung 30 steht in einem direkten Zusammenhang mit der Ausdehnung des Außenringes 32, dividiert durch die Drehung des Außenringes 32 relativ zu dem Innenring 31. Die Ausdehnung ergibt sich durch die Spaltweite zwischen dem Innenring 31 und dem Außenring 32. Eine Verdrehung und Ausdehnung des Außenringes 32 ergibt sich durch die Verdrehung der Sperrelemente 35. Die Sperrelemente 35 besitzen eine Sperrelementekontur, die durch zumindest einen, vorteilhafterweise mehrere, tangential ineinander übergehende Bogenabschnitte, vorzugsweise Kreisbogenabschnitte gebildet ist. Die Spaltweite zwischen dem Innenring 31 und dem Außenring 32 ändert sich nach der Verdrehung der Sperrelemente 35, die in Sperrrichtung durch den Reibschluss mit der Verdrehung der Gelenkachse 15 gekoppelt ist. Durch die Formgebung der Sperrelemente 35 kann das Elastizitätsverhalten um die Schwenkachse 15 beeinflusst werden, sodass sich zumindest ein, insbesondere mehrere Elastizitätsbereiche ergeben. Dabei lassen sich durch die Formgebung der Sperrelemente 35 sowohl lineare als auch nichtlineare, insbesondere progressive Federcharakteristiken realisieren. Gemäß der Figur 31 sind drei Elastizitätsbereiche vorhanden. In der Anwendung erscheint es vorteilhaft, zu Beginn einen relativ weichen Elastizitätsbereich für eine initiale Kniebeugung zu haben, um eine ausreichende Standbeugung bei niedrigeren Momenten zu ermöglichen. In dem mittleren Elastizitätsbereich könnte sich die Elastizität durch die Konturgeometrie reduzieren, um eine ausreichende Steifigkeit und Sicherheit zu ermöglichen. Im dritten Elastizitätsbereich und Endbereich ist die Kontur beispielsweise so gestaltet, dass die Sperreinrichtung bei einer vorgegebenen Last anfängt zu rutschen, um so einen Überlastschutz zu bewirken.

## Patentansprüche

1. Orthopädietechnische Gelenkeinrichtung mit einem Oberteil (10) und einem um eine Schwenkachse (15) schwenkbar daran gelagerten Unterteil (20) und einer Sperreinrichtung (30), die eine Verschwenkung des Oberteils (10) relativ zu dem Unterteil (20) sperrt, wobei die Sperreinrichtung (30) in einer Schwenkrichtung nie sperrt und in der entgegengesetzten Schwenkrichtung schaltbar aus einer Freigabestellung in eine Sperrstellung bringbar ist, wobei der Sperreinrichtung (30) ein Betätigungselement (40) zugeordnet ist, das die Sperreinrichtung (30) in der Freigabestellung hält oder in die Freigabestelle bewegt, **dadurch gekennzeichnet, dass** die Sperreinrichtung (30) mit einem Überlastschutz ausgestattet ist und zumindest ein Sperrelement (35) aufweist, das verschwenkbar oder verschieblich an dem Oberteil (10) gelagert ist und mit einem Sperrbereich (36) an dem Unterteil (20) oder umgekehrt in Eingriff bringbar ist, wobei das Sperrelement (35) so ausgebildet oder gelagert ist, dass bei einer Überlastung ein Durchrutschen erfolgt.

2. Orthopädietechnische Gelenkeinrichtung nach Anspruch 1, **dadurch gekennzeichnet, dass** dem Betätigungselement (40) ein Aktuator (50) zugeordnet ist, der das Betätigungselement (40) in der Freigabestellung hält oder in die Freigabestellung bewegt.

3. Orthopädietechnische Gelenkeinrichtung nach Anspruch 1, **dadurch gekennzeichnet, dass** das Sperrelement (35) als Sperrbügel, Sperrwinkel, Sperrkissen, Sperrkeil, Bremsbelag oder Sperrhaken ausgebildet ist, der oder das in der Sperrstellung an dem Sperrbereich (36) anliegt.

4. Orthopädietechnische Gelenkeinrichtung nach Anspruch 1 oder 3, **dadurch gekennzeichnet, dass** das Sperrelement (35) in einem reibschlüssigen Kontakt mit dem Sperrbereich (36) steht.

5. Orthopädietechnische Gelenkeinrichtung nach einem der voranstehenden Ansprüche, **dadurch gekennzeichnet, dass** das Sperrelement (35) elastisch in Richtung auf den Sperrbereich (36) vorgespannt ist.

6. Orthopädietechnische Gelenkeinrichtung nach Anspruch 5, **dadurch gekennzeichnet, dass** das Sperrelement (35) auf federnden Armen (13a) gelagert ist.

7. Orthopädietechnische Gelenkeinrichtung nach Anspruch 6, **dadurch gekennzeichnet, dass** den federnden Armen (13a) ein verstellbares Auflager (38) zugeordnet ist.

8. Orthopädietechnische Gelenkeinrichtung nach einem der voranstehenden Ansprüche, **dadurch gekennzeichnet, dass** der Sperreinrichtung (30) ein elastisches Element (60) vorgelagert ist.

9. Orthopädietechnische Gelenkeinrichtung nach Anspruch 8, **dadurch gekennzeichnet, dass** die Steifigkeit und/oder Vorspannung des elastischen Elementes (60) einstellbar ist.

10. Orthopädietechnische Gelenkeinrichtung nach Anspruch ist 9, **dadurch gekennzeichnet, dass** dem elastischen Element (60) ein verstellbares Auflager (65) zugeordnet ist.

11. Orthopädietechnische Gelenkeinrichtung nach Anspruch 1, **dadurch gekennzeichnet, dass** das Sperrelement (35) an einem Kopplungselement (70) gelagert ist, das starr oder elastisch mit dem Oberteil (10) oder dem Unterteil (20) verbunden ist.

12. Orthopädische Gelenkeinrichtung nach Anspruch 1, **dadurch gekennzeichnet, dass** der Überlastschutz für die Sperreinrichtung (30) durch ein Klemmelement (35a) realisiert wird, wodurch im Überlastfall ein paralleler Kraftschluss entsteht.

13. Orthopädietechnische Gelenkeinrichtung nach Anspruch 1, **dadurch gekennzeichnet, dass** die Sperreinrichtung (30) über eine zentrale Lagerung (120) geführt ist.

14. Orthopädietechnische Gelenkeinrichtung nach Anspruch 1, **dadurch gekennzeichnet, dass** das Oberteil (10) und/oder das Unterteil (20) in direktem Kontakt sowohl mit der zentralen Lagerung (120) als auch mit der Sperreinrichtung (30) stehen.

15. Orthopädietechnische Gelenkeinrichtung nach einem der voranstehenden Ansprüche, **dadurch gekennzeichnet, dass** die Sperreinrichtung (30) einen Außenring (32) und einen Innenring (31) aufweist, zwischen denen das zumindest eine Sperrelement (35) angeordnet ist.

16. Orthopädietechnische Gelenkeinrichtung nach Anspruch 15, **dadurch gekennzeichnet, dass** der Außenring (32) elastisch oder geschlitzt ausgebildet ist.

17. Orthopädietechnische Gelenkeinrichtung nach Anspruch 16, **dadurch gekennzeichnet, dass** die Elastizität des Außenringes (32) durch die Wahl des Materials und/oder der Wandstärke und/oder durch die Wahl des Innendurchmessers und/oder über die axiale Breite des Außenringes (32) eingestellt wird.

18. Orthopädietechnische Gelenkeinrichtung nach Anspruch 17, **dadurch gekennzeichnet, dass** der geschlitzte Außenring (32) verdrehgesichert in einem Gehäuse gelagert ist.

19. Orthopädietechnische Gelenkeinrichtung nach Anspruch 16 oder 18, **dadurch gekennzeichnet, dass** dem geschlitzten Außenring (32) eine Vorspanneinrichtung (326, 326) zugeordnet ist, um den Innenumfang des Außenringes (32) zu verändern.

20. Orthopädietechnische Gelenkeinrichtung nach einem der Ansprüche 16 bis 19, **dadurch gekennzeichnet, dass** eine Drehelastizität in Sperrrichtung über den Außenring (32) und/oder eine Geometrie der Sperrelemente (35) veränderbar ist.

21. Orthopädietechnische Gelenkeinrichtung nach Anspruch 20, **dadurch gekennzeichnet, dass** die Drehelastizität in Sperrrichtung einen nichtlinearen, insbesondere progressiven oder degressiven Federverlauf aufweist.

22. Orthopädietechnische Gelenkeinrichtung nach einem der voranstehenden Ansprüche, **dadurch gekennzeichnet, dass** das Oberteil (10) mit dem Unterteil (20) über zumindest ein Gelenk (101, 102) verschwenkbar um die Schwenkachse (15) an dem Oberteil (10) angeordnet ist, mit einem ersten und zumindest einem zweiten Aktuator (103, 105), die zwischen dem Oberteil (10) und dem Unterteil (20) angeordnet sind und ein erstes und zumindest ein zweites Einstellelement (104, 107) aufweisen, über die der Bewegungswiderstand des jeweils zugeordneten Aktuators (103, 105) gegen eine Verschwenkung des Unterteils (20) relativ zu dem Oberteil (10) um die Schwenkachse (15) einstellbar ist, mit zumindest einem Sensor (107) und einer Steuereinheit (108), die mit dem zumindest einen Sensor (107) zur Übermittlung von Sensordaten verbunden ist und das erste Einstellelement (104) und das zweite Einstellelement (106) in Abhängigkeit von Sensordaten synchronisiert steuert.

23. Orthopädietechnische Gelenkeinrichtung nach Anspruch 22, **dadurch gekennzeichnet, dass** mehr als zwei Aktuatoren (103, 105) durch eine Steuereinheit (108) synchronisiert gesteuert werden.

## Claims

1. An orthopedic joint device having an upper part (10), a lower part (20) mounted thereon so as to be pivotable about a pivot axis (15), and a blocking device (30) which blocks a pivoting movement of the upper part (10) relative to the lower part (20), wherein the blocking device (30) never blocks in one direction of pivoting and, in the opposite direction of pivoting, can be switched from a release position to a blocking position, wherein the blocking device (30) is assigned an actuation element (40) that holds the blocking device (30) in the release position or moves it to the release position, **characterized in that** the blocking device (30) is equipped with an overload protection and **in that** the blocking device (30) has at least one blocking element (35), which is mounted pivotably or slideably on the upper part (10) and can be engaged with a blocking region (36) on the lower part (20) or vice versa, wherein the blocking element (35) is designed or mounted in such a way that, in the event of an overload, a slip-through occurs.

2. The orthopedic joint device as claimed in claim 1, **characterized in that** the actuation element (40) is assigned an actuator (50), which holds the actuation element (40) in the release position or moves it to the release position.

3. The orthopedic joint device as claimed in claim 1, **characterized in that** the blocking element (35) is formed as a blocking bracket, blocking angle, blocking pad, blocking wedge, brake lining or blocking hook, which in the blocking position bears on the blocking region (36).

4. The orthopedic joint device as claimed in claim 1 or 3, **characterized in that** the blocking element (35) is in frictional contact with the blocking region (36).

5. The orthopedic joint device as claimed in any one of the preceding claims, **characterized in that** the blocking element (35) is elastically prestressed in the direction of the blocking region (36).

6. The orthopedic joint device as claimed in claim 5, **characterized in that** the blocking element (35) is mounted on resilient arms (13a).

7. The orthopedic joint device as claimed in claim 6, **characterized in that** the resilient arms (13a) are assigned an adjustable support (38).

8. The orthopedic joint device as claimed in any one of the preceding claims, **characterized in that** an elastic element (60) is mounted upstream of the blocking device (30).

9. The orthopedic joint device as claimed in claim 8, **characterized in that** the stiffness and/or prestressing of the elastic element (60) is adjustable.

10. The orthopedic joint device as claimed in claim 9, **characterized in that** the elastic element (60) is assigned an adjustable support (65).

11. The orthopedic joint device as claimed in claim 1, **characterized in that** the blocking element (35) is mounted on a coupling element (70), which is rigidly or elastically connected to the upper part (10) or the lower part (20).

12. The orthopedic joint device as claimed in claim 1, **characterized in that** the overload protection for the blocking device (30) is realized by a clamping element (35a), whereby a parallel force-fit arises in the event of an overload.

13. The orthopedic joint device as claimed in claim 1, **characterized in that** the blocking device (30) is guided via a central bearing (120).

14. The orthopedic joint device as claimed in claim 1, **characterized in that** the upper part (10) and/or the lower part (20) are in direct contact with both the central bearing (120) and the blocking device (30).

15. The orthopedic joint device as claimed in any one of the preceding claims, **characterized in that** the blocking device (30) has an outer ring (32) and an inner ring (31), between which the at least one blocking element (35) is arranged.

16. The orthopedic joint device as claimed in claim 15, **characterized in that** the outer ring (32) is elastic or slotted.

17. The orthopedic joint device as claimed in claim 16, **characterized in that** the elasticity of the outer ring (32) is adjusted through the choice of the material and/or the wall thickness and/or through the choice of the internal diameter and/or via the axial width of the outer ring (32).

18. The orthopedic joint device as claimed in claim 17, **characterized in that** the slotted outer ring (32) is mounted in a manner secure against rotation in a housing.

19. The orthopedic joint device as claimed in claim 16 or 18, **characterized in that** the slotted outer ring (32) is assigned a prestressing device (326) in order to change the inner circumference of the outer ring (32).

20. The orthopedic joint device as claimed in any one of claims 16 to 19, **characterized in that** a rotational elasticity in the blocking direction can be changed via the outer ring (32) and/or a geometry of the blocking elements (35).

21. The orthopedic joint device as claimed in claim 20, **characterized in that** the rotational elasticity in the blocking direction has a non-linear, in particular progressive or degressive spring profile.

22. The orthopedic joint device as claimed in any one of the preceding claims, **characterized in that** the upper part (10) with the lower part (20) is arranged pivotably about the pivot axis (15) on the upper part (10) via at least one joint (101, 102), with a first and at least a second actuator (103, 105), which are arranged between the upper part (10) and the lower part (20) and have a first and at least a second adjustment element (104, 107), via which the movement resistance of the respectively assigned actuator (103, 105) against a pivoting movement of the lower part (20) relative to the upper part (10) about the pivot axis (15) is adjustable, with at least one sensor (107) and a control unit (108), which is connected to the at least one sensor (107) for transmitting sensor data and controls the first adjustment element (104) and the second adjustment element (106) synchronously in accordance with sensor data.

23. The orthopedic joint device as claimed in claim 22, **characterized in that** more than two actuators (103, 105) are controlled synchronously by a control unit (108).

## Revendications

1. Dispositif d'articulation orthopédique comprenant une partie supérieure (10) et une partie inférieure (20) montée sur celle-ci de manière à pouvoir pivoter autour d'un axe de pivotement (15), ainsi qu'un dispositif de blocage (30) qui empêche la partie supérieure (10) de pivoter par rapport à la partie inférieure (20), dans lequel
dans un sens de pivotement, le dispositif de blocage (30) ne bloque jamais, et dans le sens de pivotement opposé, il peut être actionné pour passer d'une position de déblocage à une position de blocage,
un élément d'actionnement (40) est associé au dispositif de blocage (30), lequel maintient le dispositif de blocage (30) dans la position de déblocage ou le déplace vers la position de déblocage,
**caractérisé en ce que** le dispositif de blocage (30) est équipé d'une protection contre les surcharges et comporte au moins un élément de blocage (35) qui est monté sur la partie supérieure (10) de manière à pouvoir pivoter ou coulisser et qui peut venir en prise avec une zone de blocage (36) située sur la partie inférieure (20), ou inversement, l'élément de blocage (35) étant conçu ou monté de telle sorte qu'un glissement se produise en cas de surcharge.

2. Dispositif d'articulation orthopédique selon la revendication 1, **caractérisé en ce qu'**un actionneur (50) est associé à l'élément d'actionnement (40), lequel maintient l'élément d'actionnement (40) dans la position de déblocage ou le déplace vers la position de déblocage.

3. Dispositif d'articulation orthopédique selon la revendication 1, **caractérisé en ce que** l'élément de blocage (35) est conçu sous la forme d'un étrier de blocage, d'une équerre de blocage, d'un coussin de blocage, d'une cale de blocage, d'une garniture de frein ou d'un crochet de blocage, qui, en position de blocage, vient en appui contre la zone de blocage (36).

4. Dispositif d'articulation orthopédique selon la revendication 1 ou 3, **caractérisé en ce que** l'élément de blocage (35) est en contact par frottement avec la zone de blocage (36).

5. Dispositif d'articulation orthopédique selon l'une des revendications précédentes,
**caractérisé en ce que** l'élément de blocage (35) est précontraint élastiquement en direction de la zone de blocage (36).

6. Dispositif d'articulation orthopédique selon la revendication 5, **caractérisé en ce que** l'élément de blocage (35) est monté sur des bras à effet ressort (13a).

7. Dispositif d'articulation orthopédique selon la revendication 6, **caractérisé en ce qu'**un support réglable (38) est associé aux bras à effet ressort (13a).

8. Dispositif d'articulation orthopédique selon l'une des revendications précédentes,
**caractérisé en ce qu'**un élément élastique (60) est disposé en amont du dispositif de blocage (30).

9. Dispositif d'articulation orthopédique selon la revendication 8, **caractérisé en ce que** la rigidité et/ou la précontrainte de l'élément élastique (60) est réglable.

10. Dispositif d'articulation orthopédique selon la revendication 9, **caractérisé en ce qu'**un support réglable (65) est associé à l'élément élastique (60).

11. Dispositif d'articulation orthopédique selon la revendication 1, **caractérisé en ce que** l'élément de blocage (35) est monté sur un élément d'accouplement (70) qui est relié de manière rigide ou élastique à la partie supérieure (10) ou à la partie inférieure (20).

12. Dispositif d'articulation orthopédique selon la revendication 1, **caractérisé en ce que** la protection contre les surcharges pour le dispositif de blocage (30) est assurée par un élément de serrage (35a), ce qui permet d'obtenir en parallèle une liaison par adhérence, en cas de surcharge.

13. Dispositif d'articulation orthopédique selon la revendication 1, **caractérisé en ce que** le dispositif de blocage (30) est guidé par un palier central (120).

14. Dispositif d'articulation orthopédique selon la revendication 1, **caractérisé en ce que** la partie supérieure (10) et/ou la partie inférieure (20) sont en contact direct à la fois avec le palier central (120) et avec le dispositif de blocage (30).

15. Dispositif d'articulation orthopédique selon l'une des revendications précédentes,
**caractérisé en ce que** le dispositif de blocage (30) comporte une bague extérieure (32) et une bague intérieure (31), entre lesquelles est disposé ledit au moins un élément de blocage (35).

16. Dispositif d'articulation orthopédique selon la revendication 15, **caractérisé en ce que** la bague extérieure (32) est élastique ou fendue.

17. Dispositif d'articulation orthopédique selon la revendication 16, **caractérisé en ce que** l'élasticité de la bague extérieure (32) est réglée par le choix du matériau et/ou de l'épaisseur de paroi et/ou par le choix du diamètre intérieur et/ou par la largeur axiale de la bague extérieure (32).

18. Dispositif d'articulation orthopédique selon la revendication 17, **caractérisé en ce que** la bague extérieure fendue (32) est montée dans un boîtier de manière à être bloquée en rotation.

19. Dispositif d'articulation orthopédique selon la revendication 16 ou 18, **caractérisé en ce qu'**un dispositif de précontrainte (326, 326) est associé à la bague extérieure fendue (32) afin de modifier le périmètre intérieur de la bague extérieure (32).

20. Dispositif d'articulation orthopédique selon l'une des revendications 16 à 19,
**caractérisé en ce qu'**une élasticité en torsion dans le sens de blocage peut être modifiée par l'intermédiaire de la bague extérieure (32) et/ou de la géométrie des éléments de blocage (35).

21. Dispositif d'articulation orthopédique selon la revendication 20, **caractérisé en ce que** l'élasticité en torsion dans le sens de blocage présente une courbe d'élasticité non linéaire, en particulier progressive ou dégressive.

22. Dispositif d'articulation orthopédique selon l'une des revendications précédentes,
**caractérisé en ce que** la partie supérieure (10) est disposée sur la partie inférieure (20) de manière à pouvoir pivoter autour de l'axe de pivotement (15) par l'intermédiaire d'au moins une articulation (101, 102), comprenant un premier et au moins un deuxième actionneur (103, 105) qui sont disposés entre la partie supérieure (10) et la partie inférieure (20) et comportent un premier et au moins un deuxième élément de réglage (104, 107) qui permettent de régler la résistance au mouvement de l'actionneur (103, 105) respectivement associé pour s'opposer à un pivotement de la partie inférieure (20) par rapport à la partie supérieure (10) autour de l'axe de pivotement (15), comprenant au moins un capteur (107) et une unité de commande (108) qui est reliée audit au moins un capteur (107) pour la transmission de données de capteur et qui commande de manière synchronisée le premier élément de réglage (104) et le deuxième élément de réglage (106) en fonction des données de capteur.

23. Dispositif d'articulation orthopédique selon la revendication 22, **caractérisé en ce que** plus de deux actionneurs (103, 105) sont commandés de manière synchronisée par une unité de commande (108).
